# EUROPEAN PATENT APPLICATION

(11) **EP 4 361 250 A1**
(43) Date of publication of application: **01.05.2024**
(21) Application number: 22306628.3
(22) Date of filing: 27.10.2022
(51) Int. Cl.: C12N 1/06, C12N 1/16

(54) **YEAST COMPOSITION HAVING ANTIOXIDANT PROPERTIES**

(71) Applicant: Danstar Ferment AG, 6300 Zug (CH); Université de Bourgogne, 21078 Dijon (FR)
(72) Inventor: BAHUT, Florian, 31702 Blagnac (FR); GOUGEON, Régis D., 21078 Dijon (FR); NIKOLANTONAKI, Maria, 21078 Dijon (FR); SIECZKOWSKI, Nathalie, 31702 Blagnac (FR)
(74) Representative: J A Kemp LLP

(57) **Abstract**

The present disclosure provides a yeast composition enriched in at least one vitamin and/or in at least one sterol that can be used to provide antioxidant activity. The yeast composition can be used to prevent or limit the oxidation of an edible or a drinkable product. The yeast composition can be used to increase the robustness of a fermentative organism during a fermentation. The yeast composition can be used to provide a source of nutrition to a fermentative organism during a fermentation.

## Description

### BACKGROUND

*Non-Saccharomyces* are widely used in enology for their unique features compared to *Saccharomyces.* These yeasts are naturally present in the vineyard and in the must, but quickly disappear during the fermentation because of their lower ethanol tolerance. But the presence of *non-Saccharomyces* in the pre-fermentative steps impacts greatly the global quality of the final wine. Thus, the selection of specific *non-Saccharomyces* is nowadays at the heart of many research to develop efficient starters to improve wine quality.

The activity of *non-Saccharomyces* is attributed to their specific metabolism, this latter being the expression of the adaptation of these yeasts to their environment. It is thus accepted that depending on the environment, different yeasts (or even different strains) will exhibit a different metabolism. To obtain a specific reaction, or specific compounds, two leverages exist: the selection of a suitable strain and/or the optimization of its environment.

On the specific case of inactivated yeast in oenology, most of the work has been done on the selection at the strain level and the optimization of the culture conditions during the production of the biomass prior to inactivation because of the obligation to use only *Saccharomyces* species. Even if it shows great results, notably to increase the natural accumulation of glutathione (GSH) and other glutathione related compounds, there is still room to explore on non-conventional yeasts as well as other sources of antioxidants. Recently, some research already showed the variety of GSH production by non-Saccharomyces strains in culture medium and in wine.

There is a need to be provided with further yeast-derived compositions having antioxidant activity.

### SUMMARY

According to a first aspect, there is provided a yeast composition enriched in at least one vitamin and/or in at least one sterol and having antioxidant activity. In an embodiment, the yeast composition is an inactivated yeast composition. In another embodiment, the yeast composition is a yeast extract. In another embodiment, the yeast composition is a yeast autolysate. In a further embodiment, the yeast composition is a combination of at least two of an inactivated yeast composition, a yeast extract, a yeast autolysate or yeast cells walls. In a further embodiment, the yeast composition comprises the at least one vitamin. In yet a further embodiment, the at least one vitamin comprises a vitamin B. In an embodiment, the yeast composition comprises thiamine. In an embodiment, the yeast composition comprises nicotinic acid. In an embodiment, the yeast composition comprises pyridoxal. In an embodiment, the yeast composition comprises. In an embodiment, the yeast composition comprises niacinamide. In an embodiment, the yeast composition comprises pyridoxine. In an embodiment, the yeast composition comprises pantothenic acid. In an embodiment, the yeast composition comprises riboflavin. In an embodiment, the yeast composition comprises biotin. In an embodiment, the yeast composition comprises folic acid. In yet another embodiment, the yeast composition comprises one of more of thiamine, nicotinic acid, pyridoxal, niacinamide, pyridoxine, pantothenic acid, riboflavin, biotin or folic acid. In another embodiment, the yeast composition comprises the at least one sterol. In an embodiment, the yeast composition comprises squalene. In an embodiment, the yeast composition comprises zymosterol. In an embodiment, the yeast composition comprises ergosterol. In an embodiment, the yeast composition comprises lanosterol. In yet another embodiment, the yeast composition comprises one or more of squalene, zymosterol, ergosterol, or lanosterol. In an embodiment, the yeast composition comprises cysteine. In an embodiment, the yeast composition comprises glutathione (GSH). In an embodiment, the yeast composition comprises glutamyl-cysteine. In yet another embodiment, the yeast composition comprises one or more of cysteine, glutathione (GSH), or glutamyl-cysteine. In another embodiment, the yeast composition comprises at least one fatty acid. In an embodiment, the yeast composition comprises a C12:0 fatty acid. In an embodiment, the yeast composition comprises a C14:0 fatty acid. In an embodiment, the yeast composition comprises a C16:0 fatty acid. In an embodiment, the yeast composition comprises a C18:1 fatty acid. In an embodiment, the yeast composition comprises a C18:2 fatty acid. In an embodiment, the yeast composition comprises a C20:0 fatty acid. In yet another embodiment, the yeast composition comprises one or more of a C12:0, C14:0, C16:0, C18:1, C18:2, or C20:0 fatty acid. In another embodiment, the yeast composition comprises at least one carbohydrate. In an embodiment, the yeast composition comprises a glucan (such as, for example, one or more of a α-glucan, or a β-glucan). In an embodiment, the yeast composition comprises a mannan. In another embodiment, the yeast composition is obtained from at least one non-Saccharomyces yeast. In an embodiment, the at least one non-Saccharomyces yeast comprises a *Metschnikowia* sp. yeast. (such as, for example, *Metschnikowia pulcherrima).* In an embodiment, the at least one non-*Saccharomyces* yeast comprises a *Lachancea* sp. yeast (such as, for example, *Lachancea thermotolerans).* In an embodiment, the at least one non-Saccharomyces yeast comprises a *Torulaspora* sp. yeast (such as, for example, *Torulaspora delbrueckii).* In another embodiment, the at least one non-Saccharomyces yeast comprises a *Hanseniaspora* sp. yeast (such as, for example, *Hanseniaspora vineae).* In another embodiment, the at least one non-*Saccharomyces* yeast comprises a *Yarrowia* sp. yeast (such as, for example, *Yarrowia lipolytica).* In another embodiment, the at least one non-Saccharomyces yeast comprises a *Brettanomyces* sp. yeast (such as, for example, *Brettanomyces bruxellensis).* In another embodiment, the at least one non-Saccharomyces yeast comprises a *Pichia* sp. yeast. In another embodiment, the at least one non-Saccharomyces yeast comprises a *Starmerella* sp. yeast (such as, for example, *Starmerella bacillaris).* In another embodiment, the at least one *non-Saccharomyces* yeast comprises a *Kluyveromyces* sp. yeast (such as, for example, *Kluyveromyces marxinaus).* In another embodiment, the at least one *non-Saccharomyces* yeast comprises a *Candida* sp. yeast (such as, for example, *Candida zemplinina).*

According to a second aspect, there is provided a process for making a yeast composition. Broadly, the process comprises: (i) propagating a yeast in a propagation medium under conditions to obtain a propagated culture of the yeast enriched in at least one vitamin and/or at least one sterol; and (ii) formulating the propagated culture of the yeast enriched in at least one vitamin and/or at least one sterol in a yeast composition. In an embodiment, the propagation medium is supplemented with at least one vitamin, at least one mineral and/or a source of cysteine. In another embodiment, step (ii) comprises inactivating, extracting and/or lysing the propagated culture of the yeast to obtain an inactivated yeast composition, a yeast extract, or a yeast autolysate enriched in the at least one vitamin and/or the at least one sterol. In another embodiment, step (ii) comprises heating the propagated culture of the yeast to provide the inactivated yeast composition. In a further embodiment, step (ii) comprises submitting the propagated culture of the yeast to a pressure treatment to provide the inactivated yeast composition. In yet another embodiment, the pressure treatment comprises homogenizing the propagated cultured of the yeast to provide the inactivated yeast composition. In some embodiments, the yeast composition being obtained is defined as disclosed herein. In another embodiment, the yeast being propagated comprises a non-*Saccharomyces* yeast. In an embodiment, the *non-Saccharomyces* yeast comprises a *Metschnikowia* sp. yeast. (such as, for example, *Metschnikowia pulcherrima).* In an embodiment, the *non-Saccharomyces* yeast comprises a *Lachancea* sp. yeast (such as, for example, *Lachancea thermotolerans).* In an embodiment, the *non-Saccharomyces* yeast comprises a *Torulaspora* sp. yeast (such as, for example, *Torulaspora delbrueckii).* In another embodiment, the *non-Saccharomyces* yeast comprises a *Hanseniaspora* sp. yeast (such as, for example, *Hanseniaspora vineae).* In another embodiment, the at least one non-*Saccharomyces* yeast comprises a *Yarrowia* sp. yeast (such as, for example, *Yarrowia lipolytica).* In another embodiment, the at least one non-Saccharomyces yeast comprises a *Brettanomyces* sp. yeast (such as, for example, *Brettanomyces bruxellensis).* In another embodiment, the at least one non-Saccharomyces yeast comprises a *Candida* sp. yeast.

According to a third aspect, there is provided a yeast composition obtainable or obtained by the process described herein.

According to a fourth aspect, there is provided a method for preventing or limiting the oxidation of an edible or a drinkable product. Broadly, the method comprises contacting the yeast composition described herein with a component of the edible or the drinkable product so as to prevent or limit the oxidation of the component. In an embodiment, the method further comprises fermenting the component (or the edible/drinkable product comprising the component). In such embodiment, the method can comprise contacting the yeast composition with the component before, during and/or after fermentation. In an embodiment, the drinkable product is wine. In yet another embodiment, the component is a wine must. In an embodiment, the method can be used for protecting wine or must from oxidative damage.

According to a fifth aspect, there is provided a method of increasing the robustness of a fermentative organism during a fermentation. Broadly, the method comprises, prior to the fermentation, contacting the yeast composition described herein with the fermentative organism to increase the intracellular content of at least one vitamin and/or at least one sterol in the fermentative organism. In an embodiment, the method further comprises fermenting a biomass with the fermentative organism. In yet another embodiment, the fermentative organism comprises at least one of *Saccharomyces* sp., *Torulaspora* sp., *Metschnikowia* sp., *Pichia* sp., *Lachance* sp., *Starmerella* sp., *Kluyveromyces* sp, *Yarrowia* sp., *Brettanomyces* sp. or *Candida* sp. In an embodiment, the fermentative organism, prior to the fermentation, is provided in a dry form and is contacted with the yeast composition during rehydration.

According to a sixth aspect, there is provided a method of providing a source of nutrition to a fermentative organism during a fermentation. Broadly, the method comprises, prior to and/or during the fermentation, contacting the yeast composition described herein with the fermentative organism. In yet another embodiment, the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp, *Yarrowia* sp., *Brettanomyces* sp. or *Candida* sp. In some embodiments, the method comprising adding the yeast composition to a fermentation medium.

### BRIEF DESCRIPTION OF THE FIGURES

For a better understanding of the present invention, and to show how the same may be carried into effect, reference will now be made, by way of example, to the accompanying drawings in which;

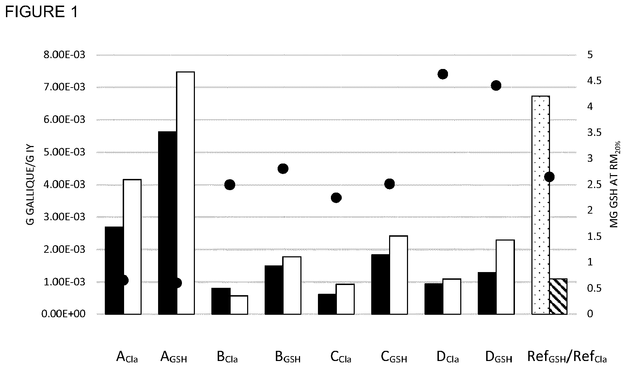

**Figure 1****:** Antioxidant capacity estimated by the DPPH assay and expressed as g of gallic acid equivalent/g of INSc (bars and left axis) and by GSH release at the Rm_{20%} (dots and right axis). The black bars correspond to INSc samples produced by thermal treatment while the white bars correspond to INSc fractions produced by high pressure homogenization. Ref represents the inactivated *Saccharomyces* samples obtained by thermal treatment (dotted bar: obtained with the glutathione culture conditions; diagonal bar: obtained with the classical culture conditions).

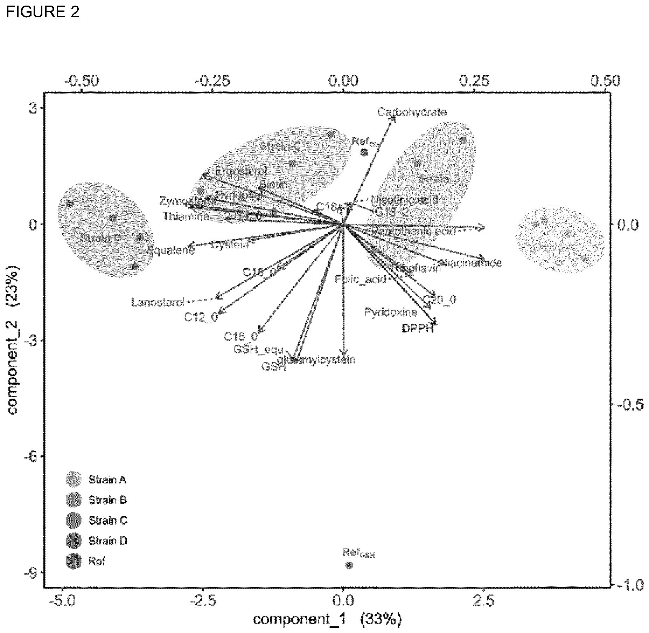

**Figure 2****:** Principal component analysis of the chemical composition of the different INSc fractions. The arrow pointing towards the DPPH score estimated the antioxidant capacity.

### DETAILED DESCRIPTION

### Yeast composition

The present disclosure provides a yeast composition. As used in the context of the present disclosure, the expression "yeast composition" refers to a composition comprises a yeast or a component derived from a yeast. It is understood that the yeast composition does not include other microbes than the yeasts or microbial components that are not derived from yeasts. The yeast composition can comprise, for example, living and active yeasts. The yeast composition can comprise, for example, semi-active yeasts. The yeast composition can comprise, for example, non-living or dead yeasts. The yeast composition can comprise components derived from a yeast (e.g., intracellular components, components of the cell membrane, components of the cell wall, as well as combination thereof). The yeast composition can be provided in a liquid, semi-liquid, or dried form.

In an embodiment, the yeast composition comprises an inactivated yeast composition. It is understood that "inactivated yeast composition" refers to a composition obtained from living yeasts which have been rendered non-viable. For example, the inactivated yeast composition can be obtained by exposing living yeasts (in embodiments, in the form of a yeast cream) to a thermal treatment and/or a pressure treatment so as to inactive the yeasts. In some specific embodiments, the inactivated yeast composition can be provided in a dried form (e.g., an inactivated dry yeast). In some embodiment, the inactivated yeast composition can be combined with living yeasts, a yeast extract, and/or a yeast autolysate.

In an embodiment, the yeast composition comprises a yeast extract. It is understood that a "yeast extract" refers to a fraction (soluble or insoluble) obtained from yeasts which have been fractionated. This fractionation can be achieved, for example, by filtration and/or centrifugation. In some embodiments, the yeasts can be submitted to a thermal (heating step or freezing step), enzymatic or pressure treatment prior to the fractionation step to generate the two fractions. In the context of the present disclosure, the yeast extract can correspond to a soluble fraction. In the context of the present disclosure, the yeast extract can correspond to an insoluble fraction (which can be referred to yeast hulls or yeast cell walls). In some specific embodiments, the yeast extract can be provided in a dried form (e.g., a dried yeast extract). In some embodiments, the yeast extract can be combined with an inactivated yeast composition, living yeasts, and/or a yeast autolysate.

In an embodiment, the yeast composition comprises a yeast autolysate. It is understood that a "yeast autolysate" refers to a composition obtained from living yeasts which have been submitted to a pH and/or thermal treatment to cause the yeast's endogenous enzymes to degrade, at least in part, the yeast components. The autolysis step can also include the addition of one or more exogenous (e.g., non-endogenous) enzymes to further degrade, at least in part, the yeasts components. Optionally, the yeast autolysate can be fractionated into a soluble and a non-soluble fraction (by filtration and/or centrifugation for example), any one of the fractions obtained can be used as the yeast autolysate. In the context of the present disclosure, the yeast autolysate can correspond to a soluble fraction. In the context of the present disclosure, the yeast autolysate can correspond to an insoluble fraction. In some specific embodiments, the yeast autolysate can be provided in a dried form (e.g., a dried yeast autolysate). In some embodiments, the yeast autolysate can be combined with an inactivated yeast composition, living yeasts, and/or a yeast extract.

In an embodiment, the yeast composition comprises living yeasts. In an embodiment, the living yeasts present in the yeast composition comprises metabolically active yeasts. The living yeast can be provided in a liquid/semi-liquid form (a cream yeast or a stabilized yeast dose for example). The living yeast can be provided in a dried form (e.g., an active dry yeast or an instant dry yeast for example).

The yeast composition of the present disclosure possesses antioxidant activity. In an embodiment, the yeast composition of the present disclosure has more antioxidant activity than a control yeast composition. The control yeast composition can be obtained, for example, from a *Saccharomyces* sp. (and in some embodiments from a *Saccharomyces cerevisiae)* that has been propagated in the absence of cysteine and glycine and generated. In such embodiment, the yeast composition and the control yeast composition can be formulated in a similar manner.

This antioxidant activity can be measured by any methods and assays known in the art and, in some embodiments, can be measured by using a 2,2-diphenyl-1-picrylhydrazyl (DPPH) assay (an embodiment of which is described in the Example). In some additional embodiments, a "yeast composition having antioxidant activity" has more than 1×10⁻³ g of gallic acid equivalent / g of the yeast composition (when measured with the DPPH assay). In some additional embodiments, a "yeast composition having antioxidant activity" has more than 1.1×10⁻³ g of gallic acid equivalent /g of the yeast composition (when measured with the DPPH assay). In some additional embodiments, a "yeast composition having antioxidant activity" has more than 1.2×10⁻³ g of gallic acid equivalent / g of the yeast composition (when measured with the DPPH assay). In some additional embodiments, a "yeast composition having antioxidant activity" has more than 1.3×10⁻³ g of gallic acid equivalent / g of the yeast composition (when measured with the DPPH assay). In some additional embodiments, a "yeast composition having antioxidant activity" has more than 1.4×10⁻³ g of gallic acid equivalent / g of the yeast composition (when measured with the DPPH assay). In some additional embodiments, a "yeast composition having antioxidant activity" has more than 1.5×10⁻³ g of gallic acid equivalent / g of the yeast composition (when measured with the DPPH assay). In some additional embodiments, a "yeast composition having antioxidant activity" has more than 1.6x10⁻³ g of gallic acid equivalent / g of the yeast composition (when measured with the DPPH assay). In some additional embodiments, a "yeast composition having antioxidant activity" has more than 1.7×10⁻³ g of gallic acid equivalent / g of the yeast composition (when measured with the DPPH assay). In some additional embodiments, a "yeast composition having antioxidant activity" has more than 1.8×10⁻³ g of gallic acid equivalent / g of the yeast composition (when measured with the DPPH assay). In some additional embodiments, a "yeast composition having antioxidant activity" has more than 1.9×10⁻³ g of gallic acid equivalent / g of the yeast composition (when measured with the DPPH assay). In some additional embodiments, a "yeast composition having antioxidant activity" has more than 2×10⁻³ g of gallic acid equivalent / g of the yeast composition (when measured with the DPPH assay).

In some embodiments, the yeast composition is enriched in at least one vitamin, e.g., the yeast composition of the present disclosure has a higher among of at least one vitamin than a control yeast composition. The control yeast composition can be obtained, for example, from a *Saccharomyces* sp. (and in some embodiments from a *Saccharomyces cerevisiae).* In some embodiments, when the yeast used to make the composition has been propagated in the presence of cysteine and glycine, the control yeast has been propagated in the presence of cysteine and glycine. In some embodiments, when the yeast used to make the composition has been propagated in the absence of cysteine and glycine, the control yeast has been propagated in the absence of cysteine and glycine and generated. In additional embodiments, the yeast composition and the control yeast composition can be formulated in a similar manner.

In an embodiment, the presence of the at least one vitamin in the yeast composition can directly provide antioxidant activity (for example by scavenging radical species). In another embodiment, the presence of the at least one vitamin can be used to generate a metabolic cofactor (coenzyme A for example), either by the yeast used to generate the yeast composition or by the fermenting organism being in contact with the yeast composition, and such metabolic cofactor could exhibit antioxidant activity. In some embodiments, providing the at least one vitamin in a yeast composition increases its bioavailability.

This enrichment in the at least one vitamin can be achieved, for example, by propagating the yeast in the presence of vitamins and/or minerals. This enrichment in the at least one vitamin can be achieved, for example, by adding the at least one vitamin during the formulation of the propagated yeast (in the yeast cream for example). This enrichment in the at least one vitamin can be achieved, for example, by selecting a yeast strain capable of accumulating the at least one vitamin more efficiently (e.g., using a yeast that is naturally enriched in the at least one vitamin), by evolving a yeast strain to accumulate the at least one vitamin more efficiently (e.g., using a yeast that is naturally enriched in the at least one vitamin) or by genetically modifying a yeast strain to accumulate the at least one vitamin more efficiently.

In an embodiment, the total amount of vitamins in the yeast composition can be equal to or higher than 23, 24, 25, 26, 27, 28, 29, 30, 31, 32, 33, 34, 35, 40, 45, 50, 55, or 60 mg / 100 g of the yeast composition. In some embodiments, the total amount of vitamins is determined on the soluble fraction associated with the yeast composition. In a specific embodiment, the total amount of vitamins in the yeast composition can be equal to or higher than 23 mg / 100 g of the yeast composition. In a specific embodiment, the total amount of vitamins in the yeast composition can be equal to or higher than 24 mg / 100 g of the yeast composition. In a specific embodiment, the total amount of vitamins in the yeast composition can be equal to or higher than 25 mg / 100 g of the yeast composition. In a specific embodiment, the total amount of vitamins in the yeast composition can be equal to or higher than 26 mg / 100 g of the yeast composition. In a specific embodiment, the total amount of vitamins in the yeast composition can be equal to or higher than 27 mg / 100 g of the yeast composition. In a specific embodiment, the total amount of vitamins in the yeast composition can be equal to or higher than 28 mg / 100 g of the yeast composition. In a specific embodiment, the total amount of vitamins in the yeast composition can be equal to or higher than 29 mg / 100 g of the yeast composition. In a specific embodiment, the total amount of vitamins in the yeast composition can be equal to or higher than 30 mg / 100 g of the yeast composition. In a specific embodiment, the total amount of vitamins in the yeast composition can be equal to or higher than 31 mg / 100 g of the yeast composition. In a specific embodiment, the total amount of vitamins in the yeast composition can be equal to or higher than 32 mg / 100 g of the yeast composition. In a specific embodiment, the total amount of vitamins in the yeast composition can be equal to or higher than 33 mg / 100 g of the yeast composition. In a specific embodiment, the total amount of vitamins in the yeast composition can be equal to or higher than 34 mg / 100 g of the yeast composition. In a specific embodiment, the total amount of vitamins in the yeast composition can be equal to or higher than 35 mg / 100 g of the yeast composition. In a specific embodiment, the total amount of vitamins in the yeast composition can be equal to or higher than 40 mg / 100 g of the yeast composition. In a specific embodiment, the total amount of vitamins in the yeast composition can be equal to or higher than 45 mg / 100 g of the yeast composition. In a specific embodiment, the total amount of vitamins in the yeast composition can be equal to or higher than 50 mg / 100 g of the yeast composition. In a specific embodiment, the total amount of vitamins in the yeast composition can be equal to or higher than 55 mg / 100 g of the yeast composition. In a specific embodiment, the total amount of vitamins in the yeast composition can be equal to or higher than 60 mg / 100 g of the yeast composition.

The at least one vitamin of the yeast composition can comprise one or more water soluble vitamin. The at least one vitamin of the yeast composition can comprise one or more fat soluble vitamin. The at least one vitamin of the yeast composition can comprise one or more water soluble vitamin and one or more fat soluble vitamin. The at least one vitamin of the yeast composition can comprise vitamin A, optionally in combination with other vitamins. The at least one vitamin of the yeast composition can comprise vitamin B, optionally in combination with other vitamins. The at least one vitamin of the yeast composition can comprise vitamin C, optionally in combination with other vitamins. The at least one vitamin of the yeast composition can comprise vitamin D, optionally in combination with other vitamins. The at least one vitamin of the yeast composition can comprise vitamin E, optionally in combination with other vitamins. The at least one vitamin of the yeast composition can comprise vitamin K, optionally in combination with other vitamins.

The at least one vitamin of the yeast composition can comprise one or more vitamin B. In an embodiment, the total amount of vitamin B in the yeast composition can be equal to or higher than 23, 24, 25, 26, 27, 28, 29, 30, 31, 32, 33, 34, 35, 40, 45, 50, 55, or 60 mg / 100 g of the yeast composition. In some embodiments, the total amount of vitamin B is determined on the soluble fraction associated with the yeast composition. In a specific embodiment, the total amount of vitamins B in the yeast composition can be equal to or higher than 23 mg / 100 g of the yeast composition. In a specific embodiment, the total amount of vitamins B in the yeast composition can be equal to or higher than 24 mg / 100 g of the yeast composition. In a specific embodiment, the total amount of vitamins B in the yeast composition can be equal to or higher than 25 mg / 100 g of the yeast composition. In a specific embodiment, the total amount of vitamins B in the yeast composition can be equal to or higher than 26 mg / 100 g of the yeast composition. In a specific embodiment, the total amount of vitamins B in the yeast composition can be equal to or higher than 27 mg / 100 g of the yeast composition. In a specific embodiment, the total amount of vitamins B in the yeast composition can be equal to or higher than 28 mg / 100 g of the yeast composition. In a specific embodiment, the total amount of vitamins B in the yeast composition can be equal to or higher than 29 mg / 100 g of the yeast composition. In a specific embodiment, the total amount of vitamins B in the yeast composition can be equal to or higher than 30 mg / 100 g of the yeast composition. In a specific embodiment, the total amount of vitamins B in the yeast composition can be equal to or higher than 31 mg / 100 g of the yeast composition. In a specific embodiment, the total amount of vitamins B in the yeast composition can be equal to or higher than 32 mg / 100 g of the yeast composition. In a specific embodiment, the total amount of vitamins B in the yeast composition can be equal to or higher than 33 mg / 100 g of the yeast composition. In a specific embodiment, the total amount of vitamins B in the yeast composition can be equal to or higher than 34 mg / 100 g of the yeast composition. In a specific embodiment, the total amount of vitamins B in the yeast composition can be equal to or higher than 35 mg / 100 g of the yeast composition. In a specific embodiment, the total amount of vitamins B in the yeast composition can be equal to or higher than 40 mg / 100 g of the yeast composition. In a specific embodiment, the total amount of vitamins B in the yeast composition can be equal to or higher than 45 mg / 100 g of the yeast composition. In a specific embodiment, the total amount of vitamins B in the yeast composition can be equal to or higher than 50 mg / 100 g of the yeast composition. In a specific embodiment, the total amount of vitamins B in the yeast composition can be equal to or higher than 55 mg / 100 g of the yeast composition. In a specific embodiment, the total amount of vitamins B in the yeast composition can be equal to or higher than 60 mg / 100 g of the yeast composition.

In an embodiment, the yeast composition comprises one of more of thiamine, nicotinic acid, pyridoxal, niacinamide, pyridoxine, pantothenic acid, riboflavin, biotin, or folic acid. In an embodiment, the at least one vitamin of the yeast composition can comprise pyridoxine, folic acid, riboflavin, niacinamide, pantothenic acid, or combinations thereof. In alternative embodiment, the at least one vitamin of the yeast composition can be substantially free from thiamine, pyridoxal, biotin and/or nicotinic acid.

In some embodiments, the at least one vitamin of the yeast composition can comprise vitamin C. Without wishing to be bound to theory, vitamin C can be used to scavenge radical species.

In some embodiments, the at least one vitamin of the yeast composition can comprise thiamine (or vitamin B1). In an embodiment, the amount of thiamine in the yeast composition can be equal to or higher than 4, 5, 6, 7, 8, 9, 10, 11, 12, 13, 14, 15, 16, 17, 18, 19, or 20 mg / 100 g of the yeast composition. In some embodiments, the total amount of thiamine is determined on the soluble fraction associated with the yeast composition. In a specific embodiment, the amount of thiamine in the yeast composition can be equal to or higher than 4 mg / 100 g of the yeast composition. In a specific embodiment, the amount of thiamine in the yeast composition can be equal to or higher than 5 mg / 100 g of the yeast composition. In a specific embodiment, the amount of thiamine in the yeast composition can be equal to or higher than 6 mg / 100 g of the yeast composition. In a specific embodiment, the amount of thiamine in the yeast composition can be equal to or higher than 7 mg / 100 g of the yeast composition. In a specific embodiment, the amount of thiamine in the yeast composition can be equal to or higher than 8 mg / 100 g of the yeast composition. In a specific embodiment, the amount of thiamine in the yeast composition can be equal to or higher than 9 mg / 100 g of the yeast composition. In a specific embodiment, the amount of thiamine in the yeast composition can be equal to or higher than 10 mg / 100 g of the yeast composition. In a specific embodiment, the amount of thiamine in the yeast composition can be equal to or higher than 11 mg / 100 g of the yeast composition. In a specific embodiment, the amount of thiamine in the yeast composition can be equal to or higher than 12 mg / 100 g of the yeast composition. In a specific embodiment, the amount of thiamine in the yeast composition can be equal to or higher than 13 mg / 100 g of the yeast composition. In a specific embodiment, the amount of thiamine in the yeast composition can be equal to or higher than 14 mg / 100 g of the yeast composition. In a specific embodiment, the amount of thiamine in the yeast composition can be equal to or higher than 15 mg / 100 g of the yeast composition. In a specific embodiment, the amount of thiamine in the yeast composition can be equal to or higher than 16 mg / 100 g of the yeast composition. In a specific embodiment, the amount of thiamine in the yeast composition can be equal to or higher than 17 mg / 100 g of the yeast composition. In a specific embodiment, the amount of thiamine in the yeast composition can be equal to or higher than 18 mg / 100 g of the yeast composition. In a specific embodiment, the amount of thiamine in the yeast composition can be equal to or higher than 19 mg / 100 g of the yeast composition. In a specific embodiment, the amount of thiamine in the yeast composition can be equal to or higher than 20 mg / 100 g of the yeast composition. In alternative embodiments, the yeast composition is substantially free from thiamine (and can have, for example, an amount equal to or less than 3 mg / 100 g of the yeast composition.

In some embodiments, the at least one vitamin of the yeast composition can comprise riboflavin (e.g., a form of vitamin B2). Without wishing to be bound to theory, riboflavin can be used to scavenge radical species. In an embodiment, the amount of riboflavin in the yeast composition can be equal to or higher than 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, or 4.0 mg / 100 g of the yeast composition. In some embodiments, the total amount of riboflavin is determined on the soluble fraction associated with the yeast composition. In a specific embodiment, the amount of riboflavin in the yeast composition can be equal to or higher than 1.3 mg / 100 g of the yeast composition. In a specific embodiment, the amount of riboflavin in the yeast composition can be equal to or higher than 1.4 mg / 100 g of the yeast composition. In a specific embodiment, the amount of riboflavin in the yeast composition can be equal to or higher than 1.5 mg / 100 g of the yeast composition. In a specific embodiment, the amount of riboflavin in the yeast composition can be equal to or higher than 1.6 mg / 100 g of the yeast composition. In a specific embodiment, the amount of riboflavin in the yeast composition can be equal to or higher than 1.7 mg / 100 g of the yeast composition. In a specific embodiment, the amount of riboflavin in the yeast composition can be equal to or higher than 1.8 mg / 100 g of the yeast composition. In a specific embodiment, the amount of riboflavin in the yeast composition can be equal to or higher than 1.9 mg / 100 g of the yeast composition. In a specific embodiment, the amount of riboflavin in the yeast composition can be equal to or higher than 2.0 mg / 100 g of the yeast composition. In a specific embodiment, the amount of riboflavin in the yeast composition can be equal to or higher than 2.1 mg / 100 g of the yeast composition. In a specific embodiment, the amount of riboflavin in the yeast composition can be equal to or higher than 2.2 mg / 100 g of the yeast composition. In a specific embodiment, the amount of riboflavin in the yeast composition can be equal to or higher than 2.3 mg / 100 g of the yeast composition. In a specific embodiment, the amount of riboflavin in the yeast composition can be equal to or higher than 2.4 mg / 100 g of the yeast composition. In a specific embodiment, the amount of riboflavin in the yeast composition can be equal to or higher than 2.5 mg / 100 g of the yeast composition. In a specific embodiment, the amount of riboflavin in the yeast composition can be equal to or higher than 2.6 mg / 100 g of the yeast composition. In a specific embodiment, the amount of riboflavin in the yeast composition can be equal to or higher than 2.7 mg / 100 g of the yeast composition. In a specific embodiment, the amount of riboflavin in the yeast composition can be equal to or higher than 2.8 mg / 100 g of the yeast composition. In a specific embodiment, the amount of riboflavin in the yeast composition can be equal to or higher than 2.9 mg / 100 g of the yeast composition. In a specific embodiment, the amount of riboflavin in the yeast composition can be equal to or higher than 3.0 mg / 100 g of the yeast composition. In a specific embodiment, the amount of riboflavin in the yeast composition can be equal to or higher than 3.1 mg / 100 g of the yeast composition. In a specific embodiment, the amount of riboflavin in the yeast composition can be equal to or higher than 3.2 mg / 100 g of the yeast composition. In a specific embodiment, the amount of riboflavin in the yeast composition can be equal to or higher than 3.3 mg / 100 g of the yeast composition. In a specific embodiment, the amount of riboflavin in the yeast composition can be equal to or higher than 3.4 mg / 100 g of the yeast composition. In a specific embodiment, the amount of riboflavin in the yeast composition can be equal to or higher than 3.5 mg / 100 g of the yeast composition. In a specific embodiment, the amount of riboflavin in the yeast composition can be equal to or higher than 3.6 mg / 100 g of the yeast composition. In a specific embodiment, the amount of riboflavin in the yeast composition can be equal to or higher than 3.7 mg / 100 g of the yeast composition. In a specific embodiment, the amount of riboflavin in the yeast composition can be equal to or higher than 3.8 mg / 100 g of the yeast composition. In a specific embodiment, the amount of riboflavin in the yeast composition can be equal to or higher than 3.9 mg / 100 g of the yeast composition. In a specific embodiment, the amount of riboflavin in the yeast composition can be equal to or higher than 4.0 mg / 100 g of the yeast composition.

In some embodiments, the at least one vitamin of the yeast composition can comprise nicotinic acid (e.g., a form of vitamin B3). In an embodiment, the amount of nicotinic acid in the yeast composition can be equal to or higher than 4.2, 4.3, 4.4, 4.5, 4.6, 4.7, or 4.8 mg / 100 g of the yeast composition. In some embodiments, the total amount of nicotinic acid is determined on the soluble fraction associated with the yeast composition. In a specific embodiment, the amount of nicotinic acid in the yeast composition can be equal to or higher than 4.2 mg / 100 g of the yeast composition. In a specific embodiment, the amount of nicotinic acid in the yeast composition can be equal to or higher than 4.3 mg / 100 g of the yeast composition. In a specific embodiment, the amount of nicotinic acid in the yeast composition can be equal to or higher than 4.4 mg / 100 g of the yeast composition. In a specific embodiment, the amount of nicotinic acid in the yeast composition can be equal to or higher than 4.5 mg / 100 g of the yeast composition. In a specific embodiment, the amount of nicotinic acid in the yeast composition can be equal to or higher than 4.6 mg / 100 g of the yeast composition. In a specific embodiment, the amount of nicotinic acid in the yeast composition can be equal to or higher than 4.7 mg / 100 g of the yeast composition. In a specific embodiment, the amount of nicotinic acid in the yeast composition can be equal to or higher than 4.8 mg / 100 g of the yeast composition. In alternative embodiments, the yeast composition is substantially free from nicotinic acid (and can have, for example, an amount less than 4.2 mg / 100 g of the yeast composition).

In some embodiments, the at least one vitamin of the yeast composition can comprise niacinamide (e.g., a form of vitamin B3). In an embodiment, the amount of niacinamide in the yeast composition can be equal to or higher than 0.01, 0.02, 0.03, 0.04, 0.05, 0.06, 0.07, 0.08, 1, 2, 3, 4, 5, 6, 7, or 8 mg / 100 g of the yeast composition. In some embodiments, the total amount of niacinamide is determined on the soluble fraction associated with the yeast composition. In a specific embodiment, the amount of niacinamide in the yeast composition can be equal to or higher than 0.01 mg / 100 g of the yeast composition. In a specific embodiment, the amount of niacinamide in the yeast composition can be equal to or higher than 0.02 mg / 100 g of the yeast composition. In a specific embodiment, the amount of niacinamide in the yeast composition can be equal to or higher than 0.03 mg / 100 g of the yeast composition. In a specific embodiment, the amount of niacinamide in the yeast composition can be equal to or higher than 0.04 mg / 100 g of the yeast composition. In a specific embodiment, the amount of niacinamide in the yeast composition can be equal to or higher than 0.05 mg / 100 g of the yeast composition. In a specific embodiment, the amount of niacinamide in the yeast composition can be equal to or higher than 0.06 mg / 100 g of the yeast composition. In a specific embodiment, the amount of niacinamide in the yeast composition can be equal to or higher than 0.07 mg / 100 g of the yeast composition. In a specific embodiment, the amount of niacinamide in the yeast composition can be equal to or higher than 0.08 mg / 100 g of the yeast composition. In a specific embodiment, the amount of niacinamide in the yeast composition can be equal to or higher than 0.09 mg / 100 g of the yeast composition. In a specific embodiment, the amount of niacinamide in the yeast composition can be equal to or higher than 1 mg / 100 g of the yeast composition. In a specific embodiment, the amount of niacinamide in the yeast composition can be equal to or higher than 2 mg / 100 g of the yeast composition. In a specific embodiment, the amount of niacinamide in the yeast composition can be equal to or higher than 3 mg / 100 g of the yeast composition. In a specific embodiment, the amount of niacinamide in the yeast composition can be equal to or higher than 4 mg / 100 g of the yeast composition. In a specific embodiment, the amount of niacinamide in the yeast composition can be equal to or higher than 5 mg / 100 g of the yeast composition. In a specific embodiment, the amount of niacinamide in the yeast composition can be equal to or higher than 6 mg / 100 g of the yeast composition. In a specific embodiment, the amount of niacinamide in the yeast composition can be equal to or higher than 7 mg / 100 g of the yeast composition. In a specific embodiment, the amount of niacinamide in the yeast composition can be equal to or higher than 8 mg / 100 g of the yeast composition.

In some embodiments, the at least one vitamin of the yeast composition can comprise pantothenic acid (or vitamin B5). Without wishing to be bound to theory, pantothenic acid is not known to exert antioxidant activity per se, however it is known to be a precursor for coenzyme A. Coenzyme A is a key metabolic intermediate in living cells. In addition, coenzyme A possesses a free sulfhydryl group, and thus could play a role in the antioxidant capacity of the yeast composition described herein. In an embodiment, the amount of pantothenic acid in the yeast composition can be equal to or higher than 7, 8, 9, 10, 11, 12, 13, 14, 15, 16, 17, 18, 19, or 20 mg / 100 g of the yeast composition. In some embodiments, the total amount of pantothenic acid is determined on the soluble fraction associated with the yeast composition. In a specific embodiment, the amount of pantothenic acid in the yeast composition can be equal to or higher than 7 mg / 100 g of the yeast composition. In a specific embodiment, the amount of pantothenic acid in the yeast composition can be equal to or higher than 8 mg / 100 g of the yeast composition. In a specific embodiment, the amount of pantothenic acid in the yeast composition can be equal to or higher than 9 mg / 100 g of the yeast composition. In a specific embodiment, the amount of pantothenic acid in the yeast composition can be equal to or higher than 10 mg / 100 g of the yeast composition. In a specific embodiment, the amount of pantothenic acid in the yeast composition can be equal to or higher than 11 mg / 100 g of the yeast composition. In a specific embodiment, the amount of pantothenic acid in the yeast composition can be equal to or higher than 12 mg / 100 g of the yeast composition. In a specific embodiment, the amount of pantothenic acid in the yeast composition can be equal to or higher than 13 mg / 100 g of the yeast composition. In a specific embodiment, the amount of pantothenic acid in the yeast composition can be equal to or higher than 14 mg / 100 g of the yeast composition. In a specific embodiment, the amount of pantothenic acid in the yeast composition can be equal to or higher than 15 mg / 100 g of the yeast composition. In a specific embodiment, the amount of pantothenic acid in the yeast composition can be equal to or higher than 16 mg / 100 g of the yeast composition. In a specific embodiment, the amount of pantothenic acid in the yeast composition can be equal to or higher than 17 mg / 100 g of the yeast composition. In a specific embodiment, the amount of pantothenic acid in the yeast composition can be equal to or higher than 18 mg / 100 g of the yeast composition. In a specific embodiment, the amount of pantothenic acid in the yeast composition can be equal to or higher than 19 mg / 100 g of the yeast composition. In a specific embodiment, the amount of pantothenic acid in the yeast composition can be equal to or higher than 20 mg / 100 g of the yeast composition.

In some embodiments, the at least one vitamin of the yeast composition can comprise pyridoxal (e.g., a form of vitamin B6). In alternative embodiments, the yeast composition is substantially free from pyridoxal acid (and can have, for example, an amount less than 0.3 mg / 100 g of the yeast composition).

In some embodiments, the at least one vitamin of the yeast composition can comprise pyridoxin (e.g., a form of vitamin B6). Without wishing to be bound to theory, pyridoxin can be used to scavenge radical species.

In some embodiments, the at least one vitamin of the yeast composition can comprise biotin (vitamin B7). In alternative embodiments, the yeast composition is substantially free from biotin (and can have, for example, an amount less than 17 µg / 100 g of the yeast composition).

In some embodiments, the at least one vitamin of the yeast composition can comprise folic acid (vitamin B9). Without wishing to be bound to theory, folic can be used to scavenge radical species.

In some embodiments, the yeast composition is enriched in at least one sterol, e.g., the yeast composition of the present disclosure has a higher among of at least sterol than a control yeast composition. The control yeast composition can be obtained, for example, from a *Saccharomyces* sp. (and in some embodiments from a *Saccharomyces cerevisiae).* In some embodiments, the control yeast has been propagated in the presence of cysteine and glycine and generated. In some embodiments, the control yeast has been propagated in the absence of cysteine and glycine and generated. In additional embodiments, the yeast composition and the control yeast composition can be formulated in a similar manner.

In an embodiment, the presence of the at least sterol in the yeast composition can directly provide antioxidant activity (for example by scavenging radical species). In another embodiment, the presence of the at sterol can be used to generate a metabolic cofactor, either by the yeast used to generate the yeast composition or by the fermenting organism being in contact with the yeast composition, and such metabolic cofactor could exhibit antioxidant activity. In some embodiments, providing the at least sterol in a yeast composition increases its bioavailability.

This enrichment in the at least one sterol can be achieved, for example, by propagating the yeast in the presence of vitamins and/or minerals. This enrichment in the at least one sterol can be achieved, for example, by adding the at least one sterol during the formulation of the propagated yeast (in the yeast cream for example). This enrichment in the at least one sterol can be achieved, for example, by selecting a yeast strain capable of accumulating the at least one sterol more efficiently, by evolving a yeast strain to increase its capacity to accumulate the at least one sterol more efficiently or by genetically modifying the yeast strain to increase its capacity to accumulate the at least one sterol more efficiently.

In an embodiment, the total amount of sterol in the yeast composition is higher than 0.2, or 0.3 mg / 100 g of the yeast composition. In a specific embodiment, the total amount of sterol in the yeast composition is equal to or higher than 0.2 mg / 100 g of the yeast composition. In a specific embodiment, the total amount of sterol in the yeast composition is equal to or higher than 0.3 mg / 100 g of the yeast composition. In some embodiments, the total amount of sterols is determined on the soluble fraction associated with the yeast composition.

The at least one sterol of the yeast composition can comprise one or more of squalene, zymosterol, ergosterol, or lanosterol. In some embodiments, the at least one sterol of the yeast composition can comprise lanosterol. In alternative embodiments, the at least one sterol of the yeast composition can be substantially free of squalene, ergosterol and/or of zymosterol.

In an embodiment, the at least one sterol comprises squalene. In alternative embodiments, the yeast composition is substantially free of squalene (and can have, for example, an amount less than 0.02 mg / 100 g of the yeast composition).

In an embodiment, the at least one sterol comprises zymosterol. In alternative embodiments, the yeast composition is substantially free of zymosterol (and can have, for example, an amount less than 0.03 mg / 100 g of the yeast composition).

In an embodiment, the at least one sterol comprises ergosterol. In alternative embodiments, the yeast composition is substantially free of ergosterol (and can have, for example, an amount less than 0.3 mg / 100 g of the yeast composition).

In an embodiment, the at least one sterol comprises lanosterol. In a specific embodiment, the yeast composition comprises a total amount of lanosterol of at least 0.01, or 0.02 mg / 100 g of composition. In some embodiments, the total amount of lanosterol is determined on the soluble fraction associated with the yeast composition. In a specific embodiment, the yeast composition comprises a total amount of lanosterol of at least 0.01 mg / 100 g of composition. In a specific embodiment, the yeast composition comprises a total amount of lanosterol of at least 0.02 mg / 100 g of composition.

In some embodiments, the yeast composition comprises a glutathione-related compound. As used in the context of the present disclosure, the expression "glutathione-related compound" refers to a metabolic precursor of glutathione (cysteine and glutamyl-cysteine for example) as well as glutathione itself (in an oxidized or reduced form). In some embodiments, the yeast composition of the present disclosure has a higher among of the at least one glutathione compound (e.g., is enriched) than a control yeast composition. The control yeast composition can be obtained, for example, from a *Saccharomyces* sp. (and in some embodiments from a *Saccharomyces cerevisiae).* In some embodiments, the control yeast has been propagated in the absence of cysteine and glycine and generated. In additional embodiments, the yeast composition and the control yeast composition can be formulated in a similar manner.

In embodiments, this enrichment in the at least one glutathione compound can be achieved, for example, by propagating the yeast in the presence of vitamins and/or minerals. This enrichment in the at least one sterol can be achieved, for example, by adding the at least one glutathione compound during the formulation of the propagated yeast (in the yeast cream for example). This enrichment in the at least one glutathione compound can be achieved, for example, by selecting a yeast strain capable of accumulating the at least one glutathione compound more efficiently, by evolving a yeast strain to increase its capacity to accumulate the at least one glutathione compound more efficiently or by genetically modifying the yeast strain to increase its capacity to accumulate the at least one glutathione compound more efficiently.

In some embodiments, the amount of glutathione compounds is provided as discrete value for each of the different compounds that may be present in the yeast composition. In an embodiment, the yeast composition comprises cysteine. For example, the yeast composition can comprise more than 0.13, 0.14, 0.15, 0.16, 0.17, 0.18, 0.19, 0.20, 0.21, 0.22, 0.23, 0.24, 0.25, 0.26, 0.27, 0.28, 0.29, or 30 mg of cysteine / g of the yeast composition. In some embodiments, the total amount of cysteine is determined on the soluble fraction associated with the yeast composition. In a specific embodiment, the yeast composition can comprise more than 0.13 mg of cysteine/g of the yeast composition. In a specific embodiment, the yeast composition can comprise an amount of cysteine equal to or higher than 0.14 mg / g of the yeast composition. In a specific embodiment, the yeast composition can comprise an amount of cysteine equal to or higher than 0.15 mg / g of the yeast composition. In a specific embodiment, the yeast composition can comprise an amount of cysteine equal to or higher than 0.16 mg / g of the yeast composition. In a specific embodiment, the yeast composition can comprise an amount of cysteine equal to or higher than 0.17 mg / g of the yeast composition. In a specific embodiment, the yeast composition can comprise an amount of cysteine equal to or higher than 0.18 mg / g of the yeast composition. In a specific embodiment, the yeast composition can comprise an amount of cysteine equal to or higher than 0.19 mg / g of the yeast composition. In a specific embodiment, the yeast composition can comprise an amount of cysteine equal to or higher than 0.20 mg / g of the yeast composition. In a specific embodiment, the yeast composition can comprise an amount of cysteine equal to or higher than 0.21 mg / g of the yeast composition. In a specific embodiment, the yeast composition can comprise an amount of cysteine equal to or higher than 0.22 mg / g of the yeast composition. In a specific embodiment, the yeast composition can comprise an amount of cysteine equal to or higher than 0.23 mg / g of the yeast composition. In a specific embodiment, the yeast composition can comprise an amount of cysteine equal to or higher than 0.24 mg / g of the yeast composition. In a specific embodiment, the yeast composition can comprise an amount of cysteine equal to or higher than 0.25 mg / g of the yeast composition. In a specific embodiment, the yeast composition can comprise an amount of cysteine equal to or higher than 0.26 mg / g of the yeast composition. In a specific embodiment, the yeast composition can comprise an amount of cysteine equal to or higher than 0.27 mg / g of the yeast composition. In a specific embodiment, the yeast composition can comprise an amount of cysteine equal to or higher than 0.28 mg / g of the yeast composition. In a specific embodiment, the yeast composition can comprise an amount of cysteine equal to or higher than 0.29 mg / g of the yeast composition. In a specific embodiment, the yeast composition can comprise an amount of cysteine equal to or higher than 0.30 mg / g of the yeast composition. In alternative embodiments, the yeast composition is substantially free from cysteine (for example the yeast composition can comprise an amount of cysteine equal to lower than 0.13 mg /g of the yeast composition).

In an embodiment, the yeast composition comprises glutathione (GSH). For example, the yeast composition can comprise more than 3.3, 3.4, 3.5, 3.6, 3.7, 3.8, 3.9, 4.0, 4.1, 4.2, 4.3, 4.4, 4.5, 4.6, 4.7, 4.8, 4.9, 5, 6, 7, 8, 9, 10, 11, 12, 13, 14, 15, 16, 17, 18, 19, 20, 21, 22, 23, 24, or 25 mg of GSH / g of the yeast composition. In some embodiments, the total amount of GSH is determined on the soluble fraction associated with the yeast composition. In a specific embodiment, the yeast composition can comprise more than 3.3 mg of GSH / g of the yeast composition. In a specific embodiment, the yeast composition can comprise an amount of GSH equal to or higher than 3.4 mg / g of the yeast composition. In a specific embodiment, the yeast composition can comprise an amount of GSH equal to or higher than 3.5 mg / g of the yeast composition. In a specific embodiment, the yeast composition can comprise an amount of GSH equal to or higher than 3.6 mg / g of the yeast composition. In a specific embodiment, the yeast composition can comprise an amount of GSH equal to or higher than 3.7 mg / g of the yeast composition. In a specific embodiment, the yeast composition can comprise an amount of GSH equal to or higher than 3.8 mg / g of the yeast composition. In a specific embodiment, the yeast composition can comprise an amount of GSH equal to or higher than 3.9 mg / g of the yeast composition. In a specific embodiment, the yeast composition can comprise an amount of GSH equal to or higher than 4.0 mg / g of the yeast composition. In a specific embodiment, the yeast composition can comprise an amount of GSH equal to or higher than 4.1 mg / g of the yeast composition. In a specific embodiment, the yeast composition can comprise an amount of GSH equal to or higher than 4.2 mg / g of the yeast composition. In a specific embodiment, the yeast composition can comprise an amount of GSH equal to or higher than 4.3 mg / g of the yeast composition. In a specific embodiment, the yeast composition can comprise an amount of GSH equal to or higher than 4.4 mg / g of the yeast composition. In a specific embodiment, the yeast composition can comprise an amount of GSH equal to or higher than 4.5 mg / g of the yeast composition. In a specific embodiment, the yeast composition can comprise an amount of GSH equal to or higher than 4.6 mg / g of the yeast composition. In a specific embodiment, the yeast composition can comprise an amount of GSH equal to or higher than 4.7 mg / g of the yeast composition. In a specific embodiment, the yeast composition can comprise an amount of GSH equal to or higher than 4.8 mg / g of the yeast composition. In a specific embodiment, the yeast composition can comprise an amount of GSH equal to or higher than 4.9 mg / g of the yeast composition. In a specific embodiment, the yeast composition can comprise an amount of GSH equal to or higher than 5 mg / g of the yeast composition. In a specific embodiment, the yeast composition can comprise an amount of GSH equal to or higher than 6 mg / g of the yeast composition. In a specific embodiment, the yeast composition can comprise an amount of GSH equal to or higher than 7 mg / g of the yeast composition. In a specific embodiment, the yeast composition can comprise an amount of GSH equal to or higher than 8 mg / g of the yeast composition. In a specific embodiment, the yeast composition can comprise an amount of GSH equal to or higher than 9 mg / g of the yeast composition. In a specific embodiment, the yeast composition can comprise an amount of GSH equal to or higher than 10 mg / g of the yeast composition. In a specific embodiment, the yeast composition can comprise an amount of GSH of at least 10 mg / g of the yeast composition. In a specific embodiment, the yeast composition can comprise an amount of GSH equal to or higher than 11 mg / g of the yeast composition. In a specific embodiment, the yeast composition can comprise an amount of GSH of at least 11 mg / g of the yeast composition. In a specific embodiment, the yeast composition can comprise an amount of GSH equal to or higher than 12 mg / g of the yeast composition. In a specific embodiment, the yeast composition can comprise an amount of GSH of at least 12 mg /g of the yeast composition. In a specific embodiment, the yeast composition can comprise an amount of GSH equal to or higher than 13 mg / g of the yeast composition. In a specific embodiment, the yeast composition can comprise an amount of GSH of at least 13 mg / g of the yeast composition. In a specific embodiment, the yeast composition can comprise an amount of GSH equal to or higher than 14 mg / g of the yeast composition. In a specific embodiment, the yeast composition can comprise an amount of GSH of at least 14 mg / g of the yeast composition. In a specific embodiment, the yeast composition can comprise an amount of GSH equal to or higher than 15 mg / g of the yeast composition. In a specific embodiment, the yeast composition can comprise an amount of GSH of at least 15 mg / g of the yeast composition. In a specific embodiment, the yeast composition can comprise an amount of GSH equal to or higher than 16 mg / g of the yeast composition. In a specific embodiment, the yeast composition can comprise an amount of GSH of at least 16 mg / g of the yeast composition. In a specific embodiment, the yeast composition can comprise an amount of GSH equal to or higher than 17 mg / g of the yeast composition. In a specific embodiment, the yeast composition can comprise an amount of GSH of at least 17 mg / g of the yeast composition. In a specific embodiment, the yeast composition can comprise an amount of GSH equal to or higher than 18 mg / g of the yeast composition. In a specific embodiment, the yeast composition can comprise an amount of GSH of at least 18 mg / g of the yeast composition. In a specific embodiment, the yeast composition can comprise an amount of GSH equal to or higher than 19 mg / g of the yeast composition. In a specific embodiment, the yeast composition can comprise an amount of GSH of at least 19 mg / g of the yeast composition. In a specific embodiment, the yeast composition can comprise an amount of GSH equal to or higher than 20 mg / g of the yeast composition. In a specific embodiment, the yeast composition can comprise an amount of GSH of at least 20 mg / g of the yeast composition. In a specific embodiment, the yeast composition can comprise an amount of GSH equal to or higher than 21 mg / g of the yeast composition. In a specific embodiment, the yeast composition can comprise an amount of GSH of at least 21 mg / g of the yeast composition. In a specific embodiment, the yeast composition can comprise an amount of GSH equal to or higher than 22 mg / g of the yeast composition. In a specific embodiment, the yeast composition can comprise an amount of GSH of at least 22 mg / g of the yeast composition. In a specific embodiment, the yeast composition can comprise an amount of GSH equal to or higher than 23 mg / g of the yeast composition. In a specific embodiment, the yeast composition can comprise an amount of GSH of at least 23 mg / g of the yeast composition. In a specific embodiment, the yeast composition can comprise an amount of GSH equal to or higher than 24 mg / g of the yeast composition. In a specific embodiment, the yeast composition can comprise an amount of GSH of at least 24 mg / g of the yeast composition. In a specific embodiment, the yeast composition can comprise an amount of GSH equal to or higher than 25 mg / g of the yeast composition. In a specific embodiment, the yeast composition can comprise an amount of GSH of at least 25 mg / g of the yeast composition. In alternative embodiments, the yeast composition is substantially free from GSH (for example the yeast composition can comprise an amount of GSH equal to lower than 3.3 mg / g of the yeast composition).

In an embodiment, the yeast composition comprises glutamyl-cysteine. For example, the yeast composition can comprise more than 0.43, 0.44, 0.45, 0.46, 0.47, 0.48, 0.49, 0.50, 1, 2, 3, 4, or 5 mg of glutamyl-cysteine / g of the yeast composition. In some embodiments, the total amount of glutamyl-cysteine is determined on the soluble fraction associated with the yeast composition. In a specific embodiment, the yeast composition can comprise more than 0.43 mg of glutamyl-cysteine / g of the yeast composition. In a specific embodiment, the yeast composition can comprise an amount of glutamyl-cysteine equal to or higher than 0.44 mg / g of the yeast composition. In a specific embodiment, the yeast composition can comprise an amount of glutamyl-cysteine equal to or higher than 0.45 mg / g of the yeast composition. In a specific embodiment, the yeast composition can comprise an amount of glutamyl-cysteine equal to or higher than 0.46 mg / g of the yeast composition. In a specific embodiment, the yeast composition can comprise an amount of glutamyl-cysteine equal to or higher than 0.47 mg / g of the yeast composition. In a specific embodiment, the yeast composition can comprise an amount of glutamyl-cysteine equal to or higher than 0.48 mg / g of the yeast composition. In a specific embodiment, the yeast composition can comprise an amount of glutamyl-cysteine equal to or higher than 0.49 mg / g of the yeast composition. In a specific embodiment, the yeast composition can comprise an amount of glutamyl-cysteine equal to or higher than 0.50 mg / g of the yeast composition. In a specific embodiment, the yeast composition can comprise an amount of glutamyl-cysteine equal to or higher than 1 mg / g of the yeast composition. In a specific embodiment, the yeast composition can comprise an amount of glutamyl-cysteine equal to or higher than 2 mg / g of the yeast composition. In a specific embodiment, the yeast composition can comprise an amount of glutamyl-cysteine equal to or higher than 3 mg / g of the yeast composition. In a specific embodiment, the yeast composition can comprise an amount of glutamyl-cysteine equal to or higher than 4 mg / g of the yeast composition. In a specific embodiment, the yeast composition can comprise an amount of glutamyl-cysteine equal to or higher than 5 mg/g of the yeast composition. In alternative embodiments, the yeast composition is substantially free from glutamyl-cysteine (for example the yeast composition can comprise an amount of glutamyl-cysteine equal to lower than 0.43 mg / g of the yeast composition).

In some embodiments, the amount of glutathione-related compounds can be provided as "glutathione (glutathione equivalent) equivalent". For example, the yeast composition can comprise more than 4.0, 4.1, 4.2, 4.3, 4.4, 4.5, 4.6, 4.7, 4.8, 4.9, 5, 6, 7, 8, 9, or 10 mg of glutathione equivalent / g of the yeast composition. In some embodiments, the total amount of glutathione equivalent is determined on the soluble fraction associated with the yeast composition. In a specific embodiment, the yeast composition can comprise an amount of glutathione equivalent equal to or higher than 4.0 mg / g of the yeast composition. In a specific embodiment, the yeast composition can comprise an amount of glutathione equivalent equal to or higher than 4.1 mg / g of the yeast composition. In a specific embodiment, the yeast composition can comprise an amount of glutathione equivalent equal to or higher than 4.2 mg /g of the yeast composition. In a specific embodiment, the yeast composition can comprise an amount of glutathione equivalent equal to or higher than 4.3 mg / g of the yeast composition. In a specific embodiment, the yeast composition can comprise an amount of glutathione equivalent equal to or higher than 4.4 mg / g of the yeast composition. In a specific embodiment, the yeast composition can comprise an amount of glutathione equivalent equal to or higher than 4.5 mg / g of the yeast composition. In a specific embodiment, the yeast composition can comprise an amount of glutathione equivalent equal to or higher than 4.6 mg /g of the yeast composition. In a specific embodiment, the yeast composition can comprise an amount of glutathione equivalent equal to or higher than 4.7 mg / g of the yeast composition. In a specific embodiment, the yeast composition can comprise an amount of glutathione equivalent equal to or higher than 4.8 mg / g of the yeast composition. In a specific embodiment, the yeast composition can comprise an amount of glutathione equivalent equal to or higher than 4.9 mg / g of the yeast composition. In a specific embodiment, the yeast composition can comprise an amount of glutathione equivalent equal to or higher than 5 mg / g of the yeast composition. In a specific embodiment, the yeast composition can comprise an amount of glutathione equivalent equal to or higher than 6 mg / g of the yeast composition. In a specific embodiment, the yeast composition can comprise an amount of glutathione equivalent equal to or higher than 7 mg / g of the yeast composition. In a specific embodiment, the yeast composition can comprise an amount of glutathione equivalent equal to or higher than 8 mg / g of the yeast composition. In a specific embodiment, the yeast composition can comprise an amount of glutathione equivalent equal to or higher than 9 mg / g of the yeast composition. In a specific embodiment, the yeast composition can comprise an amount of glutathione equivalent equal to or higher than 10 mg / g of the yeast composition. In a specific embodiment, the yeast composition can comprise an amount of glutathione equivalent of at least 10 mg / g of the yeast composition. In alternative embodiments, the yeast composition is substantially free from glutathione equivalent (for example the yeast composition can comprise an amount of glutathione equivalent equal to lower than 4.0 mg / g of the yeast composition).

In some embodiments, the yeast composition comprises the at least one fatty acid. In some further embodiments, the yeast composition is enriched in the at least one fatty acid, e.g., the yeast composition can have a higher amount (e.g., enriched) of at least one fatty acid than a control yeast composition. The control yeast composition can be obtained, for example, from a *Saccharomyces* sp. (and in some embodiments from a *Saccharomyces cerevisiae).* In some embodiments, the control yeast has been propagated in the presence of cysteine and glycine. In some embodiments, the control yeast has been propagated in the absence of cysteine and glycine and generated. In additional embodiments, the yeast composition and the control yeast composition can be formulated in a similar manner.

This enrichment in the at least one fatty acid can be achieved, for example, by propagating the yeast in the presence of vitamins and/or minerals. This enrichment in the at least one fatty acid can be achieved, for example, by adding the at least one fatty acid during the formulation of the propagated yeast (in the yeast cream for example). This enrichment in the at least one fatty acid can be achieved, for example, by selecting a yeast strain capable of accumulating the at least one fatty acid more efficiently, by evolving a yeast strain to accumulate the at least one fatty acid more efficiently or by genetically modifying a yeast strain to accumulate the at least one fatty acid more efficiently.

In an embodiment, the total amount of fatty acids in the yeast composition can be equal to or higher than 600, 700, 800, 900, 1000, 1100, 1200, 1300, or 1400 mg / 100 g of the yeast composition. In some embodiments, the total amount of fatty acids is determined on the soluble fraction associated with the yeast composition. In a specific embodiment, the total amount of fatty acids in the yeast composition can be equal to or higher than 600 mg / 100 g of the yeast composition. In a specific embodiment, the total amount of fatty acids in the yeast composition can be equal to or higher than 700 mg / 100 g of the yeast composition. In a specific embodiment, the total amount of fatty acids in the yeast composition can be equal to or higher than 800 mg / 100 g of the yeast composition. In a specific embodiment, the total amount of fatty acids in the yeast composition can be equal to or higher than 900 mg / 100 g of the yeast composition. In a specific embodiment, the total amount of fatty acids in the yeast composition can be equal to or higher than 1000 mg / 100 g of the yeast composition. In a specific embodiment, the total amount of fatty acids in the yeast composition can be equal to or higher than 1100 mg / 100 g of the yeast composition. In a specific embodiment, the total amount of fatty acids in the yeast composition can be equal to or higher than 1200 mg / 100 g of the yeast composition. In a specific embodiment, the total amount of fatty acids in the yeast composition can be equal to or higher than 1300 mg / 100 g of the yeast composition. In a specific embodiment, the total amount of fatty acids in the yeast composition can be equal to or higher than 1400 mg / 100 g of the yeast composition.

In an embodiment, the yeast composition comprises C12:0, C14:0, C16:0, C18:0, C18:1, C18:2, and/or C20:0 fatty acids, or combinations thereof. In some embodiments, the yeast composition substantially lacks C12:0, C14:0, C16:0, C18:0, C18:1, and/or C18:2 fatty acids. In another embodiment, the yeast composition comprises C20:0 fatty acids. In yet another embodiment, the yeast composition substantially lacks C6:0, C8:0 and C10:0 (e.g., above a detectable level).

In an embodiment, the yeast composition comprises C12:0 fatty acids. In alternative embodiments, the yeast composition substantially lacks C12:0 fatty acids.

In an embodiment, the yeast composition comprises C14:0 fatty acids. In alternative embodiments, the yeast composition substantially lacks C14:0 fatty acids.

In an embodiment, the yeast composition comprises C16:0 fatty acids. In alternative embodiments, the yeast composition substantially lacks C16:0 fatty acids.

In an embodiment, the yeast composition comprises C18:1 fatty acids. In alternative embodiments, the yeast composition substantially lacks C18:1 fatty acids.

In an embodiment, the yeast composition comprises C18:2 fatty acids. In alternative embodiments, the yeast composition substantially lacks C18:2 fatty acids.

In an embodiment, the yeast composition comprises C20:0 fatty acids.

In some embodiments, the yeast composition comprises at least one carbohydrate. In some further embodiments, the yeast composition is enriched in at least one carbohydrate, e.g., the yeast composition of the present disclosure can have a higher amount (e.g., enriched) of at least one carbohydrate than a control yeast composition. The control yeast composition can be obtained, for example, from a *Saccharomyces* sp. (and in some embodiments from a *Saccharomyces cerevisiae).* In some embodiments, the control yeast has been propagated in the presence of cysteine and glycine. In some embodiments, the control yeast has been propagated in the absence of cysteine and glycine and generated. In additional embodiments, the yeast composition and the control yeast composition can be formulated in a similar manner. Without wishing to be bound to theory, carbohydrates do not exert, in the composition, an antioxidant effect. As such, in some embodiments, the yeast composition can substantially lack carbohydrates.

This enrichment in the at least one carbohydrate can be achieved, for example, by propagating the yeast in the presence of vitamins and/or minerals. This enrichment in the at least one carbohydrate can be achieved, for example, by adding the at least one carbohydrate during the formulation of the propagated yeast (in the yeast cream for example). This enrichment in the at least one carbohydrate can be achieved, for example, by selecting a yeast strain capable of accumulating the at least one carbohydrate more efficiently, by evolving a yeast strain to accumulate the at least one carbohydrate more efficiently or by genetically modifying a yeast strain to accumulate the at least one carbohydrate more efficiently.

In an embodiment, the total amount of carbohydrates in the yeast composition can be equal to or higher than 24, 25, 26, 27, 28, 29, 30, 31, 32, 33, or 34 mg / 100 g of the yeast composition. In a specific embodiment, the total amount of carbohydrates in the yeast composition can be equal to or higher than 24 mg / 100 g of the yeast composition. In a specific embodiment, the total amount of carbohydrates in the yeast composition can be equal to or higher than 25 mg / 100 g of the yeast composition. In a specific embodiment, the total amount of carbohydrates in the yeast composition can be equal to or higher than 26 mg / 100 g of the yeast composition. In a specific embodiment, the total amount of carbohydrates in the yeast composition can be equal to or higher than 27 mg / 100 g of the yeast composition. In a specific embodiment, the total amount of carbohydrates in the yeast composition can be equal to or higher than 28 mg / 100 g of the yeast composition. In a specific embodiment, the total amount of carbohydrates in the yeast composition can be equal to or higher than 29 mg / 100 g of the yeast composition. In a specific embodiment, the total amount of carbohydrates in the yeast composition can be equal to or higher than 30 mg / 100 g of the yeast composition. In a specific embodiment, the total amount of carbohydrates in the yeast composition can be equal to or higher than 31 mg / 100 g of the yeast composition. In a specific embodiment, the total amount of carbohydrates in the yeast composition can be equal to or higher than 32 mg / 100 g of the yeast composition. In a specific embodiment, the total amount of carbohydrates in the yeast composition can be equal to or higher than 33 mg / 100 g of the yeast composition. In a specific embodiment, the total amount of carbohydrates in the yeast composition can be equal to or higher than 34 mg / 100 g of the yeast composition.

In an embodiment, the yeast composition comprises glucans (such as, α-glucan and/or β-glucan), mannans, or combinations thereof.

In an embodiment, the yeast composition comprises glucans. In an embodiment, the total amount of glucans in the yeast composition can be equal to or higher than 11.1, 11.2, 11.3, or 11.4 mg / 100 g of the yeast composition. In a specific embodiment, the total amount of glucans in the yeast composition can be equal to or higher than 11.1 mg / 100 g of the yeast composition. In a specific embodiment, the total amount of glucans in the yeast composition can be equal to or higher than 11.2 mg / 100 g of the yeast composition. In a specific embodiment, the total amount of glucans in the yeast composition can be equal to or higher than 11.3 mg / 100 g of the yeast composition. In a specific embodiment, the total amount of glucans in the yeast composition can be equal to or higher than 11.4 mg / 100 g of the yeast composition.

In an embodiment, the yeast composition comprises α-glucans. In an embodiment, the total amount of α-glucans in the yeast composition can be equal to or higher than 0.55, 0.60, 0.65, 0.70, 0.75, or 0.80 mg / 100 g of the yeast composition. In a specific embodiment, the total amount of α-glucans in the yeast composition can be equal to or higher than 0.55 mg / 100 g of the yeast composition. In a specific embodiment, the total amount of α-glucans in the yeast composition can be equal to or higher than 0.60 mg / 100 g of the yeast composition. In a specific embodiment, the total amount of α-glucans in the yeast composition can be equal to or higher than 0.70 mg / 100 g of the yeast composition. In a specific embodiment, the total amount of α-glucans in the yeast composition can be equal to or higher than 0.75 mg / 100 g of the yeast composition. In a specific embodiment, the total amount of α-glucans in the yeast composition can be equal to or higher than 0.80 mg / 100 g of the yeast composition.

In an embodiment, the yeast composition comprises β-glucans.

In an embodiment, the yeast composition comprises mannans. In an embodiment, the total amount of mannans in the yeast composition can be equal to or higher than 12.3, 12.4, 12.5, 12.6, 12.7, 12.8, 12.9, 13.0, 13.1, 13.2, 13.3, 13.4, 13.5, 13.65, 13.7, 13.8, 13.9, or 14.0 mg / 100 g of the yeast composition. In a specific embodiment, the total amount of mannans in the yeast composition can be equal to or higher than 12.3 mg / 100 g of the yeast composition. In a specific embodiment, the total amount of mannans in the yeast composition can be equal to or higher than 12.4 mg / 100 g of the yeast composition. In a specific embodiment, the total amount of mannans in the yeast composition can be equal to or higher than 12.5 mg / 100 g of the yeast composition. In a specific embodiment, the total amount of mannans in the yeast composition can be equal to or higher than 12.6 mg / 100 g of the yeast composition. In a specific embodiment, the total amount of mannans in the yeast composition can be equal to or higher than 12.7 mg / 100 g of the yeast composition. In a specific embodiment, the total amount of mannans in the yeast composition can be equal to or higher than 12.8 mg / 100 g of the yeast composition. In a specific embodiment, the total amount of mannans in the yeast composition can be equal to or higher than 12.9 mg / 100 g of the yeast composition. In a specific embodiment, the total amount of mannans in the yeast composition can be equal to or higher than 13.0 mg / 100 g of the yeast composition. In a specific embodiment, the total amount of mannans in the yeast composition can be equal to or higher than 13.1 mg / 100 g of the yeast composition. In a specific embodiment, the total amount of mannans in the yeast composition can be equal to or higher than 13.2 mg / 100 g of the yeast composition. In a specific embodiment, the total amount of mannans in the yeast composition can be equal to or higher than 13.3 mg / 100 g of the yeast composition. In a specific embodiment, the total amount of mannans in the yeast composition can be equal to or higher than 13.4 mg / 100 g of the yeast composition. In a specific embodiment, the total amount of mannans in the yeast composition can be equal to or higher than 13.5 mg / 100 g of the yeast composition. In a specific embodiment, the total amount of mannans in the yeast composition can be equal to or higher than 13.6 mg / 100 g of the yeast composition. In a specific embodiment, the total amount of mannans in the yeast composition can be equal to or higher than 13.7 mg / 100 g of the yeast composition. In a specific embodiment, the total amount of mannans in the yeast composition can be equal to or higher than 13.8 mg / 100 g of the yeast composition. In a specific embodiment, the total amount of mannans in the yeast composition can be equal to or higher than 13.9 mg / 100 g of the yeast composition. In a specific embodiment, the total amount of mannans in the yeast composition can be equal to or higher than 14.0 mg / 100 g of the yeast composition.

The yeast composition of the present disclosure can be obtained from a source of *Saccharomyces,* a source of *non-Saccharomyces* yeasts, or a combination thereof. Each source of yeasts that can be used to prepare the yeast composition can include one or more species of yeasts.

In an embodiment, the yeast composition is obtained from a source of *Saccharomyces* yeasts. In still another embodiment, the yeast composition is obtained from a source of *Saccharomyces* yeasts which is devoid of *non-Saccharomyces* yeasts. In another embodiment, the yeast composition is obtained from a source of *non-Saccharomyces* yeasts. In still another embodiment, the yeast composition is obtained from a source of non-*Saccharomyces* yeasts which is devoid of *Saccharomyces* yeasts. In an embodiment, the yeasts used to make the composition are considered wine yeasts, as they may be present endogenously in the must or the wine and/or can be added to a must during the wine-making process. In other embodiments, yeasts used to make the composition are not considered wine yeasts. For example, the yeasts can be brewing yeasts, biofuel yeasts, etc.

Sources of *Saccharomyces* yeasts include, without limitation, *Saccharomyces cerevisiae.* In embodiments of the yeast composition obtained from using a source of *Saccharomyces* yeasts, one or more species of *Saccharomyces* sp. can be used.

In the context of the present disclosure, *"non-Saccharomyces"* yeast refer to yeasts which belong to a different genus than *Saccharomyces* sp. In embodiments of the yeast composition obtained from using a source of *non-Saccharomyces* yeasts, one or more species of non-*Saccharomyces* sp. can be used. Embodiments of non-Saccharomyces yeasts include, without limitation, *Metschnikowia* sp. (including, but not limited to *Metschnikowia pulcherrima), Lachancea* sp. (including, but not limited to *Lachancea thermotolerans), Torulaspora* sp. (including, but not limited to *Torulaspora delbrueckii), Hanseniaspora* sp.(including, but not limited to *Hanseniaspora vineae), Yarrowia* sp.(including, but not limited to *Yarrowia lipolytica), Brettanomyces* sp. (including, but not limited to *Brettanomyces bruxellensis), Pichia* sp., *Starmerella* sp. (including, but not limited to *Starmerella bacillaris*), *Kluyveromyces* sp. (including, but not limited to *Kluyveromyces marxinaus),* and *Candida* sp. (including, but not limited to *Candida zemplinina).*

### Process of making the yeast composition

The present disclosure provides a process for making the yeast composition described herein. The process comprises propagating a yeast and formulating the propagated yeasts into the yeast composition. The enrichment in the at least one vitamin and/or the at least one sterol in the yeast composition can be achieved prior to the propagation. For example, the process can include selecting a yeast species (as well as combinations thereof), or a yeast strain (as well as combinations thereof) having an increased ability to accumulate the at least one vitamin and/or the at least one sterol (e.g., yeasts which can be considered naturally enriched in that at least one vitamin and/or the at least one sterol). In another example, the process can include evolving a yeast species (as well as combinations thereof), or a yeast strain (as well as combinations thereof) to increase its ability to accumulate the at least one vitamin and/or the at least one sterol (e.g., yeasts which can be considered naturally enriched in that at least one vitamin and/or the at least one sterol). In still another example, the process can include genetically modifying a yeast species (as well as combinations thereof), or a yeast strain (as well as combinations thereof) to increase its ability to accumulate the at least one vitamin and/or the at least one sterol. Alternatively, or in combination, the enrichment in the at least one vitamin and/or the at least one sterol in the yeast composition can be achieved during to the propagation. For example, the process can comprise providing a propagation medium comprising at least one vitamin and/or at least one mineral which will enrich the propagating yeast culture in the at least one vitamin and/or the at least one sterol. Alternatively, or in combination, the enrichment in the at least one vitamin and/or the at least one sterol in the yeast composition can be achieved after to the propagation. For example, the process can comprise adding the at least one vitamin and/or the at least one sterol to the propagated culture of the yeast (or a derivative therefrom, such as a yeast cream for example) during the formulating step to obtain the yeast composition.

The first step of the process comprises propagating a yeast in a propagation medium to obtain a propagated culture of the yeast. Those skilled in the art are cognizant of the parameters (oxygenation, temperature, etc.) that can be used to favor the accumulation of the biomass and limit the production of CO₂ and ethanol. In some embodiments, the yeasts can be propagated at a temperature between about 15 to 40°C, in some additional embodiments, at a temperature between about 25 to 35°C. During the propagation, the yeasts can be cultured by constant supply of carbon sources and nitrogen sources, as well as by an intermittent supply of essential growth factors. The carbon sources can be derived from molasses and may be sugar or beetroot molasses or a mixture thereof in variable concentrations. The nitrogen source may originate from various ammoniacal derivatives, for instance ammonium hydroxide, ammonium chloride, ammonium sulfate, ammonium phosphate, diammonium phosphate and/or crude protein extracts. The yeast propagation can be performed according to a program comprising growth rate variations. The maximum growth rates can be, for example, from 0.05/h to 0.25/h and preferably from 0.15 to 0.2/h.

Yeasts that can be propagated include, but are not limited to *Saccharomyces* sp., non-*Saccharomyces* sp. and combinations thereof. The propagation step can include propagation a single source or multiple sources of yeasts. When multiple sources of yeasts are used, they can be propagated together or separately. When multiple sources of yeast are propagated separately, they can be combined prior to and/or during the formulating step.

As indicated above, in some embodiments, the propagation medium used can be enriched in one or more of a vitamin and/or a mineral. In an embodiment, the propagation medium comprises at least one of vitamin, including, but not limited to, thiamine, pyridoxine, biotin, pantothenic acid, and nicotinamide. In a specific embodiment, the propagation medium comprises thiamine, pyridoxine, biotin, pantothenic acid and/or nicotinamide. In a specific embodiment, the propagation medium comprises thiamine, pyridoxine, biotin, pantothenic acid and nicotinamide. In an embodiment, the propagation medium comprises at least one mineral, including, but not limited to, Zn, Mg, Cu, Mn, Fe, Co, B, and Na. In a specific embodiment, the propagation medium comprises Zn, Mg, Cu, Mn, Fe, Co, B, and/or Na. In a specific embodiment, the propagation medium comprises Zn, Mg, Cu, Mn, Fe, Co, B, and Na. In some embodiments, the propagation medium can include a source of cysteine and/or a source of glycine.

The second step of the process comprises formulating the propagated culture of the yeast into a yeast composition. In embodiments in which the yeast composition comprises living cells, the formulating step can include concentrating the propagated culture of yeasts (by using, for example, centrifugation and/or filtration) to provide a yeast concentrate (like a yeast cream or a stabilized liquid yeast). The yeast concentrate can be stored, frozen and/or diluted prior to use. In other embodiments in which the yeast composition comprises living cells, the formulating step can include drying the propagated culture of yeast or the yeast concentrate (to provide, for example, active dry yeasts or instant dry yeasts). The process for making the yeast composition can also include a drying step. The drying step can include, for example, roller-drying, electrospray-drying, freeze-drying, spray-drying, lyophilization and/or fluid-bed drying. The dried yeast composition can be rehydrated prior to use.

The formulation step can include, in some embodiments, inactivating (e.g., killing) the propagated culture of yeasts or the yeasts present in the yeast concentrate. This can be achieved, for example, by submitting the yeast to a thermal treatment. In some embodiments, the inactivation step comprises a heat treatment at a temperature of at least 60°C for at least 20 minutes. In some additional embodiments, the inactivation step comprises a heat treatment at a temperature between 60°C and 70°C for a period between 20 and 30 minutes. When the formulation step includes an inactivation step, it can also include a step of determining the viability of the treated yeasts and, optionally, conducting a further step of heat treatment if deemed necessary. In some embodiments, during the heat treatment step, care is taken to preserve the activity of the at least one vitamins and/or the at least one sterols that may be present in the yeast composition.

In some embodiments, the inactivation can also be achieved, for example, by submitting the yeast to a pressure treatment (e.g., homogenization, such as, for example, using bead-milling, bead-beating and/or a high-pressure homogenization). In some embodiments, during the pressure treatment step, care is taken to preserve the activity of the at least one vitamins and/or the at least one sterols that may be present in the yeast composition.

After the yeasts have been inactivated, they can optionally be dried. The dried yeast composition can be rehydrated prior to use.

The formulation step comprising inactivated yeasts can also include, in some embodiments, adding living yeasts, a yeast extract and/or a yeast autolysate to the yeast composition.

The formulation step can include, in some embodiments, fractionating the propagated culture of yeasts. This fractionation can be achieved, for example, by filtration and/or centrifugation. In some embodiments, the propagated culture of yeasts can be submitted to a thermal (heating step or freezing step), enzymatic or pressure treatment prior to the fractionation step to generate the two (or more) fractions. In the context of the present disclosure, the yeast extract can correspond to a soluble fraction. In the context of the present disclosure, the yeast extract can correspond to an insoluble fraction (which can be referred to yeast hulls or yeast cell walls). In some specific embodiments, the yeast extract can be provided in a dried form (e.g., a dried yeast extract). The dried yeast composition can be rehydrated prior to use. The formulation step of yeast compositions comprising yeast extracts can also include, in some embodiments, adding living yeasts, an inactivated yeast and/or a yeast autolysate to the yeast composition.

The formulation step can include, in some embodiments, auto-lysing the propagated culture of yeasts. This autolysis can be achieved, for example, by submitting the yeasts to a thermal treatment, optionally in the presence of exogenous enzymes. For example, the propagated culture of yeasts may be subject to a combined heat and pH treatment for a specific amount of time (e.g., 6, 12, 18, 24, 36, 48 h or more) in order to cause the autolysis of the yeasts. In another example, the propagated culture of yeasts can be submitted to a temperature of between about 40°C to about 70°C or between about 50°C to about 60°C. The propagated culture of yeasts can be submitted to a temperature of at least about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C or 70°C. Alternatively or in combination the propagated culture of yeasts can be submitted to a temperature of no more than about 70°C, 69°C, 68°C, 67°C, 66°C, 65°C, 64°C, 63°C, 62°C, 61°C, 60°C, 59°C, 58°C, 57°C, 56°C, 55°C, 54°C, 53°C, 52°C, 51°C, 50°C, 49°C, 48°C, 47°C, 46°C, 45°C, 44°C, 43°C, 42°C, 41°C or 40°C. In another example, the propagated culture of yeasts can be submitted to a pH between about 4.0 and 8.5, between about 5.0 and 7.5, or between about 5.0 and 6.0. The propagated culture of yeasts can be submitted to a pH of at least about, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4 or 8.5. Alternatively or in combination, the propagated culture of yeasts can be submitted to a pH of no more than 8.5, 8.4, 8.3, 8.2, 8.1, 8.0, 7.9, 7.8, 7.7, 7.6, 7.5, 7.4, 7.3, 7.2, 7.1, 7.0, 6.9, 6.8, 6.7, 6.6, 6.5, 6.4, 6.3, 6.2, 6.1, 6.0, 5.9, 5.8, 5.7, 5.6, 5.5, 5.4, 5.3., 5.2, 5.1, 5.0, 4.9, 4.8, 4.7, 4.6 or 4.5. Optionally, the autolyzed yeasts can be submitted to a fractionation step to obtain an insoluble and a soluble fraction. In the context of the present disclosure, the yeast autolysate can correspond to a soluble fraction. In the context of the present disclosure, the yeast autolysate can correspond to an insoluble fraction (which can be referred to yeast hulls or yeast cell walls). In some specific embodiments, the yeast autolysate can be provided in a dried form (e.g., a dried yeast autolysate). The dried yeast composition can be rehydrated prior to use. The formulation step of yeast compositions comprising yeast autolysates can also include, in some embodiments, adding living yeasts, an inactivated yeast and/or a yeast extract to the yeast composition.

### Method of using the yeast composition

In view of the fact that the yeast composition of the present disclosure exhibit antioxidant activity, they can be used to prevent and/or limit an (unwanted) oxidation reaction. In some embodiments, this antioxidant activity can be achieved directly by one or a combination of components of the yeast composition capable of limiting oxidation reactions. In other embodiments, this antioxidant activity can be achieved indirectly by one or a combination of components of the yeast composition which facilitate or enable the formation of other components (like coenzyme A) having direct antioxidant activity.

In an embodiment, the yeast composition can be used to limit oxidation in an edible or a drinkable product. This can be achieved by contacting the yeast composition with at least one component of the edible or drinkable product. In some embodiments, this can also be achieved by including the yeast composition in the edible or drinkable product (either during its production and/or after it has been produced). In some embodiments, the method comprises fermenting with a fermenting organism (such as a fermenting yeast) the components intended to be in contact, being in contact or having been in contact with the yeast composition. In such embodiment, the yeast composition can be contacted with the component prior to, during and/or after the fermentation. Such methods can be used, for example, on an edible or a drinkable product which is susceptible to oxidation. In some embodiments, the method can be used in the preparation of a drinkable product, like wine. In such embodiments, the method be used to protect wine or must from oxidative damage. Still in such embodiments, the method can include contacting a grape must with the yeast composition (before, during and/or after the fermentation). More specifically, the yeast composition can be contacted with the grape must/wine at any time prior to bottling wine at the beginning of alcoholic fermentation, in the course of it, or even when the latter is finished. In some embodiments, the yeast composition can be contacted with the grape must/wine during aging (and in some embodiments, during barrel aging). For reasons of mere convenience, the yeast composition can be contacted with the grape must/wine at the beginning of fermentation. In some embodiments, the yeast composition can be used during a pre-fermentative stabulation step, or even when fermentation is finished. In additional embodiments, it is possible to contact the yeast composition with the fermentation medium (like grape must) prior to introducing the fermenting organism in the fermentation medium.

In some embodiments, the yeast composition can be used to provide, in wines, higher quality, a more rounded product, a fresh and fruity flavor, more stable aroma(s) and/or more stable color(s). In further embodiments, the yeast composition can be used for preventing defective ageing white wines.

In specific embodiments, the yeast composition can be used with a must obtained from white grapes. For example, the must from white grapes can be provided, but are not limited to, varieties from the following groups: Siria, Malvasia Fina, Thompson seedless, Semillon, Chenin Blanc, Loureiro, Albariño, Trajadura, Sauvignon Blanc, Verdejo, Chardonnay, Gewurtzraminer, Riesling, Aligoté, Marsanne, Roussane, Petit Manseng, Gros Manseng, Colombard, Muscat, Vermentino, Gruner Vetliner, or mixtures thereof. In additional embodiments, the yeast compositions can be used with a must obtained from red grapes. For example, the red grapes can also be provided, but are not limited to varieties from the following groups: Grenache Noir, Syrah, Cabernet Franc, Cabernet Sauvignon, Merlot, Pinot Noir, Malbec, Carignan, Alicante Bouschet, Cinsault, Petit Verdot, Gamay, Mourvèdre, Mondeuse, Trousseau, Tempranillo, Tannat, Nielluccio or mixtures thereof. In further embodiments, the yeast compositions can be used with a must obtained from a mixture of red and white grapes.

In other embodiments, the yeast composition can be used to increase the robustness of a fermentative organism during a fermentation. As used in the context of the present disclosure, the expression "increase the robustness" refer to the ability of the yeast composition to confer more robustness to a fermenting organism (like a fermenting yeast, such as, for example, a wine yeast) during fermentation, when compared to the robustness of the fermenting organism in similar conditions, but in the absence of the yeast composition. The method comprises contacting the fermenting organism with the yeast composition prior to, during and/or after the fermentation. In some embodiments, the method comprises fermenting a biomass (like a must for example) with the fermentative organism. In some embodiments, the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Candida* sp., *Hanseniaspora* sp., *Yarrowia* sp., *Brettanomyces* sp. or mixtures thereof.

In still other embodiments, the method can be used to provide a source of nutrition to a fermentative organism during a fermentation. The method comprises contacting the fermenting organism with the yeast composition prior to, during and/or after the fermentation. In some embodiments, the method comprises fermenting a biomass (like a grape must for example) with the fermentative organism. In some embodiments, the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Candida* sp., *Hanseniaspora* sp., *Yarrowia* sp., *Brettanomyces* sp. or mixtures thereof.

In some embodiments, prior to fermentation, the yeast composition can be contacted with a dried fermenting organism (for example, an active dry yeast or an instant dry yeast) which has been placed in a rehydration medium during its rehydration. During the rehydration, a water solution (which can optionally include a source of carbohydrate or even grapes) can be provided at room temperature (e.g., 20 to 30°C) or heated to a temperature between 37 and 40°C. The dried fermenting organisms can be added to the rehydration medium optionally under continuous stirring/mixing. The rehydration medium can be left standing prior to inoculating into the grape must. In some embodiments, the rehydration medium can be diluted with a grape must prior to inoculating the grape must.

### EXAMPLE

*Culture of the yeast strains.* The four strains of *non-Saccharomyces* (INSc) were produced at laboratory-scale under two different culture conditions (Table 1). In addition to these strains, yeasts from *Saccharomyces* were used as minimal and maximal references, Ref_{Cla} and Ref_{GSH}, respectively, for antioxidant capacity. Ref_{Cla} and Ref_{GSH} respectfully correspond to YD6 and YD8 described in Bahut *et al.,* 2020.

Yeast creams obtained under the "classical" conditions were obtained by culturing the yeasts at a temperature of 32°C for 16 h. The propagation medium comprised 60%(w/w) regular molasses and used 5% ammonia for feeding. A special mineral and vitamin cocktail mix (comprising ZnSO₄ ·7 H₂O, MgSO₄ ·7 H₂O, thiamine, pantothenate, vitamin B6, biotine, nicotinamide, CuSO₄·5 H₂O, MnSO₄, FeNH₄S₂O₈·12 H₂O, CoSO₄·7 H₂O, H₃BO₃, and Na₂MoO₄·2 H₂O) as well as phosphoric acid 85% were added during inoculation. The propagations were carried out in lab scale bioreactor (total volume of 20 L) Sartorius (F9-I), with an aeration rate of 20 L/min and an agitation rate of 800rpm. 400 g of seed cream at 30% dry weight was used as inoculum. Sulfuric acid (2M) was used to control the pH at 6.

Yeast creams obtained under the "glutathione" conditions were obtained by culturing the yeasts as indicated above for the classical conditions, except that a cysteine and glycine solution was fed at the targeted concentration of 9 g/kg of yeast at 30% solids (Y30) and 3.6 g/kg Y30 respectively.

**Table 1: Strains and associated codes used in the Example**

| **Sample Code** | **Yeast species** | **Culture conditions** |
|---|---|---|
| A_{Cla} | *Metschnikowia pulcherrima* | Classical |
| A_{GSH} | *Metschnikowia pulcherrima* | Glutathione |
| B_{Cla} | *Lachancea thermotolerans* | Classical |
| B_{GSH} | *Lachancea thermotolerans* | Glutathione |
| C_{Cla} | *Torulaspora delbrueckii* | Classical |
| C_{GSH} | *Torulaspora delbrueckii* | Glutathione |
| D_{Cla} | *Hanseniaspora vineae* | Classical |
| D_{GSH} | *Hanseniaspora vineae* | Glutathione |
| Ref_{Cla} | *Saccharomvces cerevisiae* | Classical |
| Ref_{GSH} | *Saccharomvces cerevisiae* | Glutathione |

*Inactivation of the cream of the cultured yeast strains.* The cream obtained from cultured yeasts (irrespective of their culture conditions) were inactivated either by applying a heat treatment ("thermal" in Tables 2 to 8) or homogenization ("HPH" in Tables 2 to 8).

In the thermal treatment, approximately 500 g of a yeast cream was heated up on a programmable stirring heat plate to 70°C for 25 minutes. The inactivated yeasts were dried in a B290 Buchi mini spray dryer with an inlet temperature of 175°C (or 170°C in the case of strain A) and with a targeted outlet temperature around 86-93°C.

In the homogenization treatment, between 1 to 2 kg of a yeast cream was homogenized in batch mode using a high-pressure homogenizer (EmulsiFlex C-55, Avestin) at a homogenizing pressure of 1000 to 1500 bar. At the outlet of the homogenizer, the homogenate was cooled to ~10-15°C using a heat-exchanger connected to a chiller with recirculating water at 4°C. The homogenate was transferred back in the feed vessel and homogenized again, for a total of five passes. The inactivated yeasts were dried in a B290 Buchi mini spray dryer with an inlet temperature of 175°C (or 170°C in the case of strain A) and with a targeted outlet temperature around 86-93°C.

*Insoluble*/*soluble separation.* Inactivated yeasts were mixed in model wine solution (12% (v/v) ethanol solution, 0.01% (v/v) formic acid, pH 3.2) previously deoxygenated (bubbling argon for 15 min). After 1 h of contact with gentle and constant rotation, sample are centrifuged at 12 000*g* during 10 min at 10°C. The insoluble part was discarded, and only soluble fraction is used for further analysis, notably antioxidant capacity.

*Antioxidant capacity: DPPH assay.* The DPPH assay was performed following the protocol previously described in Romanet *et al.,* 2019. A solution of 2,2-diphenyl-1-picrylhydrazyl (DPPH) was prepared by mixing 27 mg of DPPH with 1L of 60:40 (v/v) 0.3 M buffer citrate-phosphate:methanol to reach a pH of 3.6. In absence of oxygen, 3.9 mL of the DPPH solution were mixed with 0.1 mL of sample at different mass ratio YD/DPPH (Rm). After 4 h incubation in darkness, sample absorbance was measured in a UV-Vis spectrometer at 525 nm. Absorbance was normalized with the blank (buffer with 0.1 mL of model wine). Results were expressed as the Rm needed to reduce the initial absorbance by 20%, noted Rm_{20%} and translated into the equivalent YD mass to reach the Rm_{20%}. Gallic acid was analyzed in the same conditions to express the antioxidant capacity of YD in gallic acid equivalent (GAE).

*Metabolites analysis.* In addition to antioxidant capacity analysis, the different fractions were analyzed by the Center of Food and Fermentation Technologies (Tallinn, Estonia) for solubility, glutathione and precursors, fatty acids, sterols, total carbohydrate, glucans/mannans, and vitamins.

*INSc antioxidant capacity.* The DPPH assay is a relatively quick way to estimate the antioxidant capacity of a solution. At this point it is important to clarify what is called "antioxidant capacity", it refers the antioxidant capacity of a solution refers to all the physical and chemical processes which lead to reduce the formation or the presence of oxidative compounds. In that way, consumption of oxygen, oxidant-trapping capacity or oxidant reduction capacity are all considered as characteristics that improve the antioxidant capacity.

The DPPH assay is mainly dedicated to monitor the capacity of a solution to trap or reduce radical species. Indeed, DPPH is a stable radical with a different maximum of absorption in it reduce form. It enables to follow the quantity of DPPH reduce in function of the quantity of yeast derivatives added (or other kind of sample).

The DPPH assay was applied to all the INSc samples to estimate the antioxidant capacity related to the antiradical activity. Figure 1 represents the estimated antioxidant capacity express on grams of gallic acid equivalent (GAE) per gram of INSc sample. Results can be divided in 2 parts: (1) influence of the nutrition and (2) influence of the strains.

*Culture conditions.* They are dedicated to improve the glutathione content during the biomass production and have an impact on the antioxidant capacity of all the strains. The samples with specific culture conditions ("glutathione") dedicated to accumulate GSH show a better antioxidant capacity than the "classical", except for the strain B. The difference of results between the two culture conditions is lower for *non-Saccharomyces* than for *Saccharomyces.* Without wishing to be bound to theory, it could indicate that the culture conditions do not promote GSH production in *non-Saccharomyces* to the same extend and thus have a limited impact on the antioxidant capacity, contrary to *Saccharomyces* strains, where the specific culture conditions ("glutathione") lead to a higher antioxidant capacity related to GSH itself but also co-accumulated compounds.

*Strains.* The reference *Saccharomyces* sample is the most antioxidant, but it is also interesting to see that INSc from strain A is at least 4 times better than all others INSc fractions. In addition, it is also the strain with the lowest GSH concentration released. Which clearly indicates that most of the antioxidant capacity of this strain is related to non GSH compounds. The three other strains look quite similar in antioxidant capacity.

The open dots correspond to the quantity of GSH released by the INSc fraction at the Rm_{20%} and give a comparative between the strains. This value is calculated using the grams of GSH released per grams of INSc fraction multiplied by the mass of INSc fraction needed to reach the Rm_{20%}. It allows to point out the relative importance of the GSH in the antioxidant capacity of the different INSc fraction. For example, sample CGSH and sample DGSH show a similar antioxidant capacity, while sample CGSH release two times less GSH. It clearly shows that antioxidant capacity is poorly drove by GSH. The other good example is the samples from strain A and Ref which released same amount of GSH between the two cultures condition but with a huge difference in antioxidant capacity. In this case the nutrition promotes the expression of non-GSH metabolites which could explain antioxidant capacity. It demonstrated the need to look beyond GSH when we talk about antioxidant capacity.

*Metabolites which drive antioxidant capacity.* A characterization of the insoluble fraction (Table 2), of glutathione and its precursors (Table 3), of fatty acids (Table 4), of sterols (Table 5), of total carbohydrates (Table 6), of glucans and mannans (Table 7) as well as of vitamins (Table 8) of the various samples was undertook.

**Table 2: Characterization of the insoluble fraction of the different samples tested in function of their method of inactivation. Results (provided as % of g / 100 g of the composition) are provided as an average of two replicates for each sample (except for D_{Cla} in which n: 1). SD standard deviation; HPH high pressure homogenization; NA not available.**

| | | Insoluble fraction | |
|---|---|---|---|
| **Sample** code | Inactivation | % | SD |
| A_{Cla} | Thermal | 64.9 | 0.1 |
| A_{Cla} | HPH | 60.8 | 0.3 |
| A_{GSH} | Thermal | 66.0 | 0.0 |
| A_{GSH} | HPH | 63.5 | 0.1 |
| B_{Cla} | Thermal | 68.4 | 0.1 |
| B_{Cla} | HPH | 69.1 | 0.5 |
| B_{GSH} | Thermal | 67.8 | 2.2 |
| B_{GSH} | HPH | 67.9 | 0.1 |
| C_{Cla} | Thermal | 69.4 | 0.1 |
| C_{Cla} | HPH | 68.9 | 0.6 |
| C_{GSH} | Thermal | 66.6 | 0.3 |
| C_{GSH} | HPH | 65.9 | 0.4 |
| D_{Cla} | Thermal | 65.8 | NA |
| D_{Cla} | HPH | 62.2 | 0.7 |
| D_{GSH} | Thermal | 66.3 | 0.1 |
| D_{GSH} | HPH | 62.4 | 0.5 |
| Ref_{Cla} | Thermal | 69.5 | 0.2 |
| Ref_{GSH} | Thermal | 65.3 | 0.6 |

**Table 3: Characterization of the glutathione and associated metabolites of the different samples tested in function of their method of inactivation. Results (mg / g of the composition) are provided as an average of three replicates for each sample. GSH glutathione; SD standard deviation;* under limit of detection ; ND not detected.**

| | | Cysteine | | GSH | | Glutamyl-Cysteine | | GSH equivalent | |
|---|---|---|---|---|---|---|---|---|---|
| Sample code | Inactivation | mg/g | SD | mg/g | SD | mg/g | SD | mg/g | SD |
| A_{Cla} | Thermal | ND | | 2.06 | 0.02 | 0.08 | 0.00 | 2.15 | 0.02 |
| A_{Cla} | HPH | >LOD * | | 3.20 | 0.01 | 0.04 | 0.06 | 3.28 | 0.06 |
| A_{GSH} | Thermal | >LOD * | | 4.73 | 0.09 | 0.28 | 0.00 | 5.13 | 0.10 |
| A_{GSH} | HPH | >LOD * | | 4.33 | 0.07 | 0.16 | 0.00 | 4.48 | 0.30 |
| B_{Cla} | Thermal | ND | | 1.07 | 0.02 | 0.29 | 0.00 | 1.43 | 0.02 |
| B_{Cla} | HPH | >LOD * | | 2.57 | 0.20 | 0.49 | 0.03 | 3.20 | 0.24 |
| B_{GSH} | Thermal | 0.40 | 0.01 | 4.87 | 0.06 | 1.00 | 0.01 | 7.12 | 0.09 |
| B_{GSH} | HPH | 0.27 | 0.04 | 5.88 | 1.24 | 1.80 | 0.23 | 8.17 | 1.71 |
| C_{Cla} | Thermal | ND | | 1.74 | 0.09 | 0.07 | 0.01 | 1.82 | 0.10 |
| C_{Cla} | HPH | >LOD * | | 2.28 | 0.12 | 0.09 | 0.01 | 2.39 | 0.12 |
| C_{GSH} | Thermal | 0.60 | 0.04 | 6.25 | 0.36 | 0.48 | 0.02 | 8.35 | 0.47 |
| C_{GSH} | HPH | 0.21 | 0.00 | 6.06 | 0.04 | 0.59 | 0.01 | 7.31 | 0.05 |
| D_{Cla} | Thermal | 0.24 | 0.02 | 4.73 | 0.25 | 0.18 | 0.01 | 5.57 | 0.31 |
| D_{Cla} | HPH | 0.26 | 0.05 | 6.39 | 1.19 | 0.27 | 0.05 | 7.39 | 1.39 |
| D_{GSH} | Thermal | 0.27 | 0.04 | 7.98 | 1.12 | 0.23 | 0.04 | 8.95 | 1.27 |
| D_{GSH} | HPH | 0.28 | 0.03 | 9.53 | 0.95 | 0.45 | 0.05 | 10.78 | 1.09 |
| Ref_{Cla} | Thermal | 0.13 | 0.00 | 3.26 | 0.02 | 0.43 | 0.01 | 4.01 | 0.19 |
| Ref_{GSH} | Thermal | 0.18 | 0.03 | 20.95 | 0.34 | 5.31 | 0.07 | 27.90 | 0.50 |

**Table 4: Characterization of the fatty acids of the different samples tested in function of their method of inactivation. Results are average from three replicates. Saturated fatty acids from C6:0 up to C10:0 were not detected in the samples. There are traces of C12:0, but as the peak is very small staying under limit of detection value, then the value is not accurate. SD standard deviation; * under limit of detection.**

| | | C12:0 | | C14:0 | | C16:0 | | C18:0 | | C18:1 | | C18:2 | | C20:0 | | Sum |
|---|---|---|---|---|---|---|---|---|---|---|---|---|---|---|---|---|
| Sample code | Inactivation | mg/100g | SD | mg/100g | SD | mg/100g | SD | mg/100g | SD | mg/100g | SD | mg/100g | SD | mg/100g | SD | mg/100g |
| A_{Cla} | Thermal | >LOD * | | 9.77 | 2.07 | 320.73 | 17.0 | 81.96 | 1.95 | 381.46 | 76.6 | 188.35 | 10.45 | 31.68 | 1.7 | 1013.95 |
| A_{Cla} | HPH | >LOD * | | 9.87 | 0.57 | 24.52 | 4.78 | 56.11 | 7.25 | 99.63 | 1.59 | 59.23 | 1.53 | 13.84 | 2.60 | 263.21 |
| A_{GSH} | Thermal | >LOD * | | 0.55 | 0.14 | 18.56 | 2.55 | 14.89 | 3.04 | 205.47 | 12.40 | 4.61 | 0.63 | 11.92 | 2.35 | 256.01 |
| A_{GSH} | HPH | >LOD * | | 0.60 | 0.17 | 14.31 | 2.79 | 17.13 | 1.69 | 98.56 | 4.23 | 9.56 | 1.86 | 6.56 | 1.23 | 146.73 |
| B_{Cla} | Thermal | >LOD * | | 1.49 | 0.35 | 14.41 | 1.84 | 27.68 | 2.07 | 118.59 | 14.47 | 11.11 | 3.35 | 11.50 | 2.31 | 184.78 |
| B_{Cla} | HPH | >LOD * | | 12.65 | 1.11 | 12.63 | 1.09 | 109.15 | 10.17 | 662.46 | 76.27 | 138.86 | 8.15 | 7.83 | 2.4 | 943.59 |
| B_{GSH} | Thermal | >LOD * | | 0.34 | 0.08 | 12.68 | 2.58 | 18.19 | 1.81 | 107.20 | 17.56 | 27.28 | 2.56 | ND | | 165.69 |
| B_{GSH} | HPH | >LOD * | | 33.66 | 8.7 | 9.14 | 103.5 | 200.82 | 42.2 | 586.07 | 57.1 | 302.52 | 45.8 | 6.41 | 1.6 | 1138.62 |
| C_{Cla} | Thermal | >LOD * | | 1.07 | 0.62 | 9.84 | 1.58 | 14.7 | 0.71 | 110.88 | 23.65 | 18.26 | 1.66 | 2.48 | 0.34 | 157.20 |
| C_{Cla} | HPH | 1.21 | 0.4 | 22.20 | 1.63 | 225.91 | 28.1 | 113.34 | 13.2 | 374.90 | 23.2 | 173.77 | 19.92 | 10.10 | 0.6 | 920.23 |
| C_{GSH} | Thermal | 0.84 | 0.43 | 4.58 | 2.19 | 99.29 | 8.50 | 64.33 | 3.03 | 332.20 | 43.72 | 136.54 | 4.9 | 3.70 | 1.0 | 640.64 |
| C_{GSH} | HPH | 0.55 | 0.2 | 6.20 | 1.37 | 274.76 | 31.6 | 102.35 | 15.0 | 345.19 | 27.1 | 142.75 | 11.69 | 6.19 | 0.0 | 877.44 |
| D_{Cla} | Thermal | 3.5 | 0.4 | 73.9 | 9.5 | 208.3 | 5.1 | 96.3 | 18.3 | 272.8 | 35.3 | 32.3 | 4.5 | 1.8 | 0.5 | 685.29 |
| D_{Cla} | HPH | 3.7 | 0.7 | 59.6 | 3.0 | 365.0 | 123.6 | 87.8 | 16.1 | 295.6 | 39.6 | 31.3 | 4.0 | 2.5 | 0.6 | 841.75 |
| D_{GSH} | Thermal | 3.2 | 0.6 | 42.2 | 2.7 | 612.8 | 129.5 | 71.3 | 17.9 | 266.7 | 35.3 | 26.4 | 4.6 | 1.7 | 0.3 | 1021.16 |
| D_{GSH} | HPH | 2.8 | 0.4 | 7.4 | 2.7 | 842.6 | 142.7 | 105.6 | 19.2 | 233.8 | 40.0 | 27.4 | 4.8 | 2.1 | 0.5 | 1218.96 |
| Ref_{Cla} | Thermal | 1.33 | 0.97 | 5.45 | 1.16 | 32.84 | 9.97 | 79.61 | 3.96 | 459.24 | 36.79 | 10.96 | 4.28 | 4.65 | 1.49 | 592.75 |
| Ref_{GSH} | Thermal | 4.98 | 0.6 | 5.38 | 0.47 | 1011.85 | 176.9 | 120.92 | 11.0 | 243.11 | 19.2 | 3.60 | 0.59 | 26.45 | 3.6 | 1411.31 |

**Table 5: Characterization of the sterols of the different samples tested in function of their method of inactivation. Results ( provided as % of g / g of the composition) are provided as an average of three replicates for each sample. SD standard deviation; ND compound not detected.**

| | | Squalene | | Zymosterol | | Ergosterol | | Lanosterol | | Sum |
|---|---|---|---|---|---|---|---|---|---|---|
| Sample code | Inactivation | % | SD | % | SD | % | SD | % | SD | % |
| A_{Cla} | Thermal | ND | | 0.01 | 0.00 | 0.46 | 0.03 | 0.01 | 0.00 | 0.47 |
| A_{Cla} | HPH | ND | | 0.00 | 0.00 | 0.21 | 0.00 | 0.01 | 0.00 | 0.22 |
| A_{GSH} | Thermal | ND | | 0.00 | 0.00 | 0.32 | 0.01 | 0.01 | 0.00 | 0.33 |
| A_{GSH} | HPH | ND | | 0.00 | 0.00 | 0.17 | 0.00 | 0.01 | 0.00 | 0.18 |
| B_{Cla} | Thermal | ND | | 0.01 | 0.00 | 0.34 | 0.05 | 0.01 | 0.00 | 0.36 |
| B_{Cla} | HPH | ND | | 0.01 | 0.00 | 0.40 | 0.05 | 0.01 | 0.00 | 0.42 |
| B_{GSH} | Thermal | ND | | 0.01 | 0.00 | 0.47 | 0.04 | 0.01 | 0.00 | 0.50 |
| B_{GSH} | HPH | ND | | 0.01 | 0.00 | 0.40 | 0.05 | 0.01 | 0.00 | 0.42 |
| C_{Cla} | Thermal | 0.01 | 0.00 | 0.05 | 0.00 | 0.48 | 0.01 | 0.01 | 0.00 | 0.55 |
| C_{Cla} | HPH | 0.01 | 0.00 | 0.05 | 0.00 | 0.40 | 0.03 | 0.01 | 0.00 | 0.46 |
| C_{GSH} | Thermal | 0.02 | 0.00 | 0.06 | 0.00 | 0.52 | 0.03 | 0.01 | 0.00 | 0.61 |
| C_{GSH} | HPH | 0.02 | 0.01 | 0.06 | 0.00 | 0.52 | 0.06 | 0.01 | 0.00 | 0.62 |
| D_{Cla} | Thermal | 0.05 | 0.00 | 0.05 | 0.00 | 0.65 | 0.03 | 0.02 | 0.00 | 0.77 |
| D_{Cla} | HPH | 0.04 | 0.00 | 0.05 | 0.00 | 0.64 | 0.01 | 0.02 | 0.00 | 0.74 |
| D_{GSH} | Thermal | 0.02 | 0.00 | 0.05 | 0.00 | 0.53 | 0.03 | 0.02 | 0.00 | 0.61 |
| D_{GSH} | HPH | 0.02 | 0.01 | 0.06 | 0.00 | 0.57 | 0.07 | 0.02 | 0.00 | 0.68 |
| Ref_{Cla} | Thermal | 0.01 | 0.00 | 0.03 | 0.01 | 0.28 | 0.03 | 0.01 | 0.00 | 0.33 |
| Ref_{GSH} | Thermal | 0.02 | 0.00 | 0.02 | 0.00 | 0.19 | 0.01 | 0.02 | 0.00 | 0.25 |

**Table 6: Characterization of the carbohydrates of the different samples tested in function of their method of inactivation. Results (provided as % of g / 100 g of composition) are average from three replicates. SD standard deviation.**

| | | Tot. Carbohydrate | |
|---|---|---|---|
| Sample code | Inactivation | % | SD |
| A_{Cla} | Thermal | 36.09 | 1.12 |
| A_{Cla} | HPH | 34.13 | 1.75 |
| A_{GSH} | Thermal | 38.59 | 1.70 |
| A_{GSH} | HPH | 33.57 | 2.94 |
| B_{Cla} | Thermal | 42.41 | 1.08 |
| B_{Cla} | HPH | 37.63 | 0.72 |
| B_{GSH} | Thermal | 39.86 | 2.35 |
| B_{GSH} | HPH | 32.26 | 0.60 |
| C_{Cla} | Thermal | 34.77 | 3.54 |
| C_{Cla} | HPH | 32.24 | 2.60 |
| C_{GSH} | Thermal | 32.63 | 2.41 |
| C_{GSH} | HPH | 32.06 | 1.86 |
| D_{Cla} | Thermal | 32.63 | 1.83 |
| D_{Cla} | HPH | 34.60 | 0.11 |
| D_{GSH} | Thermal | 37.86 | 2.25 |
| D_{GSH} | HPH | 31.22 | 1.15 |
| Ref_{Cla} | Thermal | 42.26 | 2.19 |
| Ref_{GSH} | Thermal | 23.02 | 3.11 |

**Table 7: Characterization of the glucans and mannans of the different samples tested in function of their method of inactivation. Results (provided as % of g / 100 g of composition) are average from three replicates. SD standard deviation.**

| | | Total glucans | | α-glucans | | β-glucans | Mannans | |
|---|---|---|---|---|---|---|---|---|
| Sample code | Inactivation | % | SD | % | SD | % | % | SD |
| A_{Cla} | Thermal | 12.14 | 0.19 | 2.15 | 0.11 | 10.00 | 14.03 | 0.27 |
| A_{Cla} | HPH | 11.54 | 0.97 | 0.87 | 0.03 | 10.67 | 14.71 | 0.76 |
| A_{GSH} | Thermal | 13.20 | 0.37 | 2.01 | 0.08 | 11.19 | 14.92 | 0.30 |
| A_{GSH} | HPH | 12.16 | 0.35 | 1.44 | 0.06 | 10.72 | 14.69 | 0.47 |
| B_{Cla} | Thermal | 19.64 | 1.13 | 5.06 | 0.30 | 14.58 | 12.95 | 0.91 |
| B_{Cla} | HPH | 17.05 | 0.64 | 3.89 | 0.20 | 13.16 | 12.58 | 0.37 |
| B_{GSH} | Thermal | 16.34 | 0.21 | 1.30 | 0.08 | 15.04 | 14.36 | 0.30 |
| B_{GSH} | HPH | 15.83 | 0.83 | 1.37 | 0.04 | 14.46 | 14.00 | 0.60 |
| C_{Cla} | Thermal | 18.26 | 0.53 | 5.21 | 0.47 | 13.05 | 13.93 | 0.43 |
| C_{Cla} | HPH | 17.26 | 0.87 | 6.71 | 0.25 | 10.55 | 13.50 | 0.71 |
| C_{GSH} | Thermal | 14.43 | 0.48 | 1.83 | 0.06 | 12.60 | 13.94 | 0.51 |
| C_{GSH} | HPH | 11.19 | 0.02 | 1.41 | 0.12 | 9.78 | 13.23 | 1.35 |
| D_{Cla} | Thermal | 12.49 | 0.29 | 0.24 | 0.02 | 12.25 | 14.67 | 0.27 |
| D_{Cla} | HPH | 11.01 | 0.26 | 0.22 | 0.01 | 10.79 | 14.09 | 0.44 |
| D_{GSH} | Thermal | 19.12 | 0.48 | 0.36 | 0.01 | 18.76 | 15.33 | 0.44 |
| D_{GSH} | HPH | 16.21 | 0.54 | 0.26 | 0.03 | 15.94 | 15.31 | 0.57 |
| Ref_{Cla} | Thermal | 27.91 | 0.63 | 5.19 | 0.26 | 22.72 | 12.93 | 0.46 |
| Ref_{GSH} | Thermal | 11.43 | 0.13 | 0.54 | 0.03 | 10.89 | 12.82 | 0.13 |

**Table 8: Characterization of the vitamins (mg / 100 g, except for biotin and folic acid provided as µg / 100g) of the different samples tested in function of their method of inactivation. Results average from three replicates. SD standard deviation.**

| | | Thiamine (B1) | | Nicotinic acid (B3) | | Pyridoxal | | Niacinamide | | Pyridoxine (B6) | | Pantothenic acid (B5) | | Riboflavin (B2) | | Biotin | | Folic acid | | Sum |
|---|---|---|---|---|---|---|---|---|---|---|---|---|---|---|---|---|---|---|---|---|
| Sample code | Inactiv. | | SD | | SD | | SD | | SD | | SD | | SD | | SD | | SD | | SD | |
| A_{Cla} | Thermal | 29.71 | 1.63 | 4.85 | 0.34 | 0.25 | 0.02 | 16.63 | 1.46 | 0.52 | 0.02 | 22.05 | 1.04 | 4.36 | 0.17 | 19.41 | 0.21 | 13.08 | 0.00 | 78.4 |
| A_{Cla} | HPH | 28.11 | 1.95 | 11.71 | 0.28 | 0.34 | 0.02 | 8.57 | 0.20 | 0.45 | 0.01 | 22.08 | 0.67 | 4.18 | 0.16 | 16.75 | 0.54 | 12.73 | 0.95 | 75.5 |
| A_{GSH} | Thermal | 19.73 | 0.99 | 4.26 | 0.02 | 0.23 | 0.01 | 15.17 | 0.53 | 0.83 | 0.03 | 19.22 | 0.66 | 4.05 | 0.28 | 13.59 | 1.26 | 6.83 | 2.38 | 63.5 |
| A_{GSH} | HPH | 25.11 | 0.87 | 7.74 | 0.51 | 0.40 | 0.03 | 8.97 | 0.38 | 0.74 | 0.05 | 20.32 | 1.06 | 4.36 | 0.27 | 14.85 | 1.65 | 14.99 | 1.80 | 67.7 |
| B_{Cla} | Thermal | 19.06 | 1.06 | 7.31 | 1.01 | 0.11 | 0.01 | 11.19 | 0.88 | 0.27 | 0.01 | 7.31 | 0.30 | 0.64 | 0.03 | 18.05 | 1.45 | 8.61 | 8.61 | 45.9 |
| B_{Cla} | HPH | 34.13 | 0.75 | 10.03 | 0.14 | 0.29 | 0.02 | 6.21 | 0.44 | 0.29 | 0.02 | 8.79 | 0.25 | 2.93 | 0.18 | 18.18 | 0.17 | 10.64 | 1.54 | 62.7 |
| B_{GSH} | Thermal | 30.34 | 1.36 | 2.37 | 0.16 | 0.33 | 0.01 | 13.65 | 0.82 | 0.69 | 0.03 | 4.54 | 0.22 | 0.73 | 0.05 | 20.25 | 2.73 | 14.89 | 0.13 | 52.7 |
| B_{GSH} | HPH | 24.42 | 1.32 | 7.01 | 0.61 | 0.44 | 0.03 | 1.37 | 0.23 | 0.80 | 0.05 | 4.84 | 0.32 | 0.89 | 0.08 | 16.03 | 0.86 | 15.13 | 0.12 | 39.8 |
| C_{Cla} | Thermal | 80.93 | 1.10 | 3.84 | 0.19 | 7.40 | 0.65 | 0.10 | 0.01 | 0.28 | 0.02 | 7.49 | 0.45 | 0.83 | 0.00 | 18.88 | 1.68 | 9.96 | 1.87 | 100.9 |
| C_{Cla} | HPH | 102.44 | 6.65 | 9.08 | 0.26 | 3.04 | 0.25 | 0.16 | 0.00 | 0.13 | 0.01 | 7.59 | 0.28 | 0.78 | 0.01 | 16.36 | 0.80 | 6.17 | 1.18 | 123.2 |
| C_{GSH} | Thermal | 109.96 | 3.93 | 1.55 | 0.02 | 10.10 | 0.78 | 0.10 | 0.00 | 0.47 | 0.01 | 3.77 | 0.12 | 0.78 | 0.02 | 24.43 | 0.52 | 8.41 | 0.25 | 126.8 |
| C_{GSH} | HPH | 116.15 | 0.52 | 6.12 | 0.23 | 1.62 | 0.10 | 0.42 | 0.01 | 0.22 | 0.00 | 3.55 | 0.23 | 0.63 | 0.03 | 15.89 | 2.25 | 12.06 | 1.41 | 128.8 |
| D_{Cla} | Thermal | 154.15 | 3.28 | 8.58 | 0.57 | 17.01 | 1.62 | 0.15 | 0.01 | 0.34 | 0.01 | 5.51 | 0.31 | 3.13 | 0.20 | 24.10 | 2.24 | 9.03 | 2.60 | 188.9 |
| D_{Cla} | HPH | 165.14 | 7.52 | 15.30 | 0.91 | 5.50 | 0.49 | 0.18 | 0.02 | 0.25 | 0.00 | 5.31 | 0.10 | 0.75 | 0.12 | 18.71 | 3.17 | 9.87 | 1.29 | 192.5 |
| D_{GSH} | Thermal | 192.61 | 5.97 | 1.25 | 0.06 | 14.16 | 0.97 | 0.07 | 0.01 | 0.36 | 0.01 | 3.48 | 0.16 | 1.90 | 0.08 | 17.95 | 1.00 | 10.79 | 0.83 | 213.9 |
| D_{GSH} | HPH | 219.31 | 9.27 | 5.51 | 0.14 | 3.99 | 0.15 | 0.07 | 0.00 | 0.12 | 0.00 | 4.27 | 0.16 | 0.17 | 0.14 | 15.96 | 1.24 | 7.48 | 2.06 | 233.5 |
| Ref_{Cla} | Thermal | 3.44 | 0.31 | 4.15 | 0.23 | 4.68 | 0.20 | 0.08 | 0.01 | 0.01 | 0.00 | 9.17 | 0.67 | 1.24 | 0.09 | 17.22 | 1.07 | 9.21 | 0.22 | 22.8 |
| Ref_{GSH} | Thermal | 3.48 | 0.15 | 4.10 | 0.38 | 0.36 | 0.01 | 10.33 | 0.47 | 0.86 | 0.03 | 7.11 | 0.28 | 2.92 | 0.10 | 16.48 | 0.34 | 13.18 | 0.63 | 29.2 |

Since the antioxidant capacity is not fully supported by the concentration of glutathione, a chemical analysis was used to explore potential relationship between metabolites and DPPH scores. Analysis of GSH and its precursors, fatty acids, sterols, carbohydrate composition, glucans/mannans composition and vitamins were integrated in a principal component analysis in addition to the DPPH results (Figure 2). Because of the different scales for all the analysis, data were centered and reduced before analysis.

Firstly, a good chemical proximity was observed between samples from the same strain. It is expected that culture conditions have a lower impact on compounds released than the strain itself. The only exception is for the reference samples from *Saccharomyces.* But these samples (from 2 different strains and 2 different production process) have been chosen for their wide differences.

On the other hand, the arrows represent the metabolites (or variables) used to produce the principal component analysis. Arrow in the same direction represents compounds which are strongly related together, for example GSH and Glutamyl-Cystein. Arrow with same direction but different orientation is negatively related (Niacinamide and Biotin). And finally, orthogonal arrows show independence of the variables (Carbohydrate and Ergosterol).

It was observed that the relative orientation of the DPPH arrow seemed to be correlated with the GSH and precursors plus some vitamins (Folic acid, Niacinamide, pantothenic acid...). Without being bound to theory, this suggests that a good DPPH score is either because of high concentration of GSH and related precursors or because of high concentrations of these vitamins.

Since all the strain are mostly aligned in the x-axis, and GSH/precursors were directed in the y-axis (mostly driven by Ref_{GSH}), it indicated that INSc antioxidant capacity (or DPPH score) is mostly driven by the composition in vitamins and not really by the concentration on GSH. It is already known that some vitamins have great antioxidant property notably to scavenge radical species (Vitamin C, Pyridoxine, Folic acid) in eucaryotic cells. The production of some of these vitamins could be an adaptative evolution for *non-Saccharomyces* to prevent oxidative damage. It could explain the antioxidant capacity of Strain A independently of the GSH concentration released.

As shown in this Example, eight inactivated non-Saccharomyces inactivated yeasts were successfully produced at lab-scale. Half of these inactivated yeasts resulting of a culture condition dedicated to accumulate glutathione, the other inactivated yeasts are produced with classical culture conditions, and all were thermally inactivated.

The different soluble fractions of each inactivated yeast were submitted to DPPH assay to estimate the antioxidant capacity. It revealed that Strain A is (from far) the most antioxidant strain. From the process point of view, the culture conditions dedicated to improving GSH always lead to better antioxidant capacity, but not directly related to GSH itself. Principal component analysis clearly showed that DPPH scores (and thus antioxidant capacity) was mainly supported by glutathione in *Saccharomyces,* and by vitamins in *non-Saccharomyces.*

Besides the use of inactivated *Saccharomyces* in oenology, there is a growing interest for non-*Saccharomyces* yeast. Their unique metabolism is already used in winemaking, but this study points out the interest of inactivated forms of non-Saccharomyces. Their unique composition, notably in vitamins, demonstrate several promising applications: in protecting must or wine from oxidative damage, protecting fermentative yeast during rehydration in order to better face the stress caused by alcoholic fermentation stresses, providing nutrition of fermentative yeasts in depleted medium.

### REFERENCES

R. Romanet, C. Coelho, Y. Liu, F. Bahut, J. Ballester, M. Nikolantonaki, R.D. Gougeon, The Antioxidant Potential of White Wines Relies on the Chemistry of Sulfur-Containing Compounds: An Optimized DPPH Assay, Molecules. 24 (2019) 1353.

Florian Bahut, Rémy Romanet, Nathalie Sieczkowski, Philippe Schmitt-Kopplin, Maria Nikolantonaki, Régis D. Gougeon, Antioxidant activity from inactivated yeast: Expanding knowledge beyond the glutathione-related oxidative stability of wine, Food Chemistry, Volume 325, 2020, 126941.

Further aspects and embodiments of the present invention are provided in the following numbered paragraphs:
1. A yeast composition enriched in at least one vitamin and/or in at least one sterol and having antioxidant activity.
2. The yeast composition of paragraph 1 being an inactivated yeast composition, a yeast extract, a yeast autolysate, or a combination thereof.
3. The yeast composition of paragraph 1 or 2 comprising the at least one vitamin.
4. The yeast composition of any one of paragraphs 1 to 3, wherein the at least one vitamin comprises a vitamin B.
5. The yeast composition of any one of paragraphs 1 to 4 comprising one of more of thiamine, nicotinic acid, pyridoxal, niacinamide, pyridoxine, pantothenic acid, riboflavin, biotin, or folic acid.
6. The yeast composition of any one of paragraphs 1 to 5 comprising the at least one sterol.
7. The yeast composition of any one of paragraphs 1 to 6 comprising one or more of squalene, zymosterol, ergosterol, or lanosterol.
8. The yeast composition of any one of paragraphs 1 to 7 comprising one or more of cysteine, glutathione (GSH), or glutamyl-cysteine.
9. The yeast composition of any one of paragraphs 1 to 8 comprising at least one fatty acid.
10. The yeast composition of any one of paragraphs 1 to 9 comprising one or more of a C12:0, C14:0, C16:0, C18:1, C18:2, or C20:0 fatty acid.
11. The yeast composition of any one of paragraphs 1 to 10 comprising at least one carbohydrate.
12. The yeast composition of any one of paragraphs 1 to 11 comprising a glucan.
13. The yeast composition of any one of paragraphs 1 to 12 comprising one or more of a α-glucan, or a β-glucan.
14. The yeast composition of any one of paragraphs 1 to 13 comprising a mannan.
15. The yeast composition of any one of paragraphs 1 to 14 being obtained from at least one non-Saccharomyces yeast.
16. The yeast composition of paragraph 15, wherein the at least one *non-Saccharomyces* yeast comprises a *Metschnikowia* sp. yeast.
17. The yeast composition of paragraph 15 or 16, wherein the at least one non-*Saccharomyces* yeast comprises *Metschnikowia pulcherrima.*
18. The yeast composition of any one of paragraphs 15 to 17, wherein the at least one *non-Saccharomyces* yeast comprises a *Lachancea* sp. yeast.
19. The yeast composition of any one of paragraphs 15 to 18, wherein the at least one *non-Saccharomyces* yeast comprises *Lachancea thermotolerans.*
20. The yeast composition of any one of paragraphs 15 to 19, wherein the at least one *non-Saccharomyces* yeast comprises a *Torulaspora* sp. yeast.
21. The yeast composition of any one of paragraphs 15 to 20, wherein the at least one *non-Saccharomyces* yeast comprises *Torulaspora delbrueckii.*
22. The yeast composition of any one of paragraphs 15 to 21, wherein the at least one *non-Saccharomyces* yeast comprises a *Hanseniaspora* sp. yeast.
23. The yeast composition of any one of paragraphs 15 to 22, wherein the at least one *non-Saccharomyces* yeast comprises *Hanseniaspora vineae.*
24. The yeast composition of any one of paragraphs 15 to 23, wherein the at least one *non-Saccharomyces* yeast comprises a *Yarrowia* sp. yeast.
25. The yeast composition of any one of paragraphs 15 to 24, wherein the at least one *non-Saccharomyces* yeast comprises *Yarrowia* lipolytica.
26. The yeast composition of any one of paragraphs 15 to 25, wherein the at least one *non-Saccharomyces* yeast comprises a *Brettanomyces* sp. yeast.
27. The yeast composition of any one of paragraphs 15 to 26, wherein the at least one *non-Saccharomyces* yeast comprises *Brettanomyces bruxellensis.*
28. The yeast composition of any one of paragraphs 15 to 27, wherein the at least one *non-Saccharomyces* yeast comprises a *Pichia* sp. yeast.
29. The yeast composition of any one of paragraphs 15 to 28, wherein the at least one *non-Saccharomyces* yeast comprises a *Starmerella* sp. yeast.
30. The yeast composition of any one of paragraphs 15 to 29, wherein the at least one *non-Saccharomyces* yeast comprises *Starmerella bacillaris.*
31. The yeast composition of any one of paragraphs 15 to 30, wherein the at least one *non-Saccharomyces* yeast comprises a *Kluyveromyces* sp. yeast.
32. The yeast composition of any one of paragraphs 15 to 31, wherein the at least one *non-Saccharomyces* yeast comprises *Kluyveromyces marxinaus.*
33. The yeast composition of any one of paragraphs 15 to 32, wherein the at least one *non-Saccharomyces* yeast comprises a *Candida* sp. yeast.
34. The yeast composition of any one of paragraphs 15 to 33, wherein the at least one *non-Saccharomyces* yeast comprises *Candida zemplinina.*
35. A process for making a yeast composition, the process comprises:
   (i) propagating a yeast in a propagation medium under conditions to obtain a propagated culture of the yeast enriched in at least one vitamin and/or at least one sterol; and
   (ii) formulating the propagated culture of the yeast enriched in at least one vitamin and/or at least one sterol in a yeast composition.
36. The process of paragraph 35, wherein the propagation medium is supplemented with at least one vitamin, at least one mineral and/or a source of cysteine.
37. The process of paragraph 35 or 36, wherein step (ii) comprises inactivating, extracting and/or lysing the propagated culture of the yeast to obtain an inactivated yeast composition, a yeast extract, or a yeast autolysate enriched in the at least one vitamin and/or the at least one sterol.
38. The process of paragraph 37, wherein step (ii) comprises heating the propagated culture of the yeast to provide the inactivated yeast composition.
39. The process of paragraph 37 or 38, wherein step (ii) comprises submitting the propagated culture of the yeast to a pressure treatment to provide the inactivated yeast composition.
40. The process of paragraph 39, wherein the pressure treatment comprises homogenizing the propagated cultured of the yeast to provide the inactivated yeast composition.
41. The process of any one of paragraphs 35 to 40, wherein the yeast composition is defined in any one of paragraphs 1 to 14.
42. The process of any one of paragraphs 35 to 41, wherein the yeast comprises a non-*Saccharomyces* yeast.
43. The process of paragraph 42, wherein the *non-Saccharomyces* yeast is defined in any one of paragraphs 16 to 34.
44. A yeast composition obtainable or obtained by the process of any one of paragraphs 35 to 43.
45. A method for preventing or limiting the oxidation of an edible or a drinkable product, the method comprising contacting the yeast composition defined in any one of paragraphs 1 to 34 and 44 with a component of the edible or the drinkable product so as to prevent or limit the oxidation of the component.
46. The method of paragraph 45, further comprising fermenting the component.
47. The method of paragraph 46, comprising contacting the yeast composition with the component before, during and/or after fermentation.
48. The method of paragraph 45 to 47, wherein the drinkable product is wine.
49. The method of paragraph 48, wherein the component is a wine must.
50. The method of paragraph 48 or 49 for protecting wine or must from oxidative damage.
51. A method of increasing the robustness of a fermentative organism during a fermentation, the method comprises, prior to the fermentation, contacting the yeast composition defined in any one of paragraphs 1 to 34 and 44 with the fermentative organism to increase the intracellular content of at least one vitamin and/or at least one sterol in the fermentative organism.
52. The method of paragraph 51, further comprising fermenting a biomass with the fermentative organism.
53. The method of paragraph 51 or 52, wherein the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Yarrowia* sp., *Brettanomyces* sp. or *Candida* sp.
54. The method of any one of paragraphs 51 to 53, wherein the fermentative organism, prior to the fermentation, is provided in a dried form and is contacted with the yeast composition during rehydration.
55. A method of providing a source of nutrition to a fermentative organism during a fermentation, the method comprises, prior to and/or during the fermentation, contacting the yeast composition defined in any one of paragraphs 1 to 34 and 44 with the fermentative organism.
56. The method of paragraph 55, wherein the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Yarrowia* sp., *Brettanomyces* sp. or *Candida* sp.
57. The method of paragraph 55 or 56 comprising adding the yeast composition to a fermentation medium.

## Claims

1. A yeast composition enriched in at least one vitamin and/or in at least one sterol and having antioxidant activity.

2. The yeast composition of claim 1, wherein:
(a) the yeast composition is an inactivated yeast composition, a yeast extract, a yeast autolysate, or a combination thereof;
(b) the yeast composition comprises the at least one vitamin;
(c) the at least one vitamin comprises a vitamin B;
(d) the yeast composition comprises one of more of thiamine, nicotinic acid, pyridoxal, niacinamide, pyridoxine, pantothenic acid, riboflavin, biotin, or folic acid;
(e) the yeast composition comprises the at least one sterol;
(f) the yeast composition comprises one or more of squalene, zymosterol, ergosterol, or lanosterol;
(g) the yeast composition comprises one or more of cysteine, glutathione (GSH), or glutamyl-cysteine;
(h) the yeast composition comprises at least one fatty acid;
(i) the yeast composition comprises one or more of a C12:0, C14:0, C16:0, C18:1, C18:2, or C20:0 fatty acid;
(j) the yeast composition comprises at least one carbohydrate;
(k) the yeast composition comprises a glucan;
(l) the yeast composition comprises one or more of a α-glucan, or a β-glucan; and/or
(m) the yeast composition comprises a mannan.

3. The yeast composition of claim 1 or 2 being obtained from at least one non-*Saccharomyces* yeast.

4. The yeast composition of claim 3, wherein the at least one non-Saccharomyces yeast comprises:
(a) a *Metschnikowia* sp. yeast;
(b) *Metschnikowia pulcherrima;*
(c) a *Lachancea* sp. yeast;
(d) *Lachancea thermotolerans;*
(e) a *Torulaspora* sp. yeast;
(f) *Torulaspora delbrueckii;*
(g) a *Hanseniaspora* sp. yeast;
(h) *Hanseniaspora vineae;*
(i) a *Yarrowia* sp. yeast;
(j) *Yarrowia lipolytica;*
(k) a *Brettanomyces* sp. yeast;
*(l) Brettanomyces bruxellensis;*
(m) a *Pichia* sp. yeast;
(n) a *Starmerella* sp. yeast;
(o) *Starmerella bacillaris;*
(p) a *Kluyveromyces* sp. yeast;
(q) *Kluyveromyces marxinaus;*
(r) *Candida* sp. yeast; and/or
(s) *Candida zemplinina.*

5. A process for making a yeast composition, the process comprises:
(i) propagating a yeast in a propagation medium under conditions to obtain a propagated culture of the yeast enriched in at least one vitamin and/or at least one sterol; and
(ii) formulating the propagated culture of the yeast enriched in at least one vitamin and/or at least one sterol in a yeast composition.

6. The process of claim 5, wherein the propagation medium is supplemented with at least one vitamin, at least one mineral and/or a source of cysteine.

7. The process of claim 5 or 6, wherein step (ii) comprises:
(a) inactivating, extracting and/or lysing the propagated culture of the yeast to obtain an inactivated yeast composition, a yeast extract, or a yeast autolysate enriched in the at least one vitamin and/or the at least one sterol;
(b) heating the propagated culture of the yeast to provide the inactivated yeast composition; and/or
(c) submitting the propagated culture of the yeast to a pressure treatment to provide the inactivated yeast composition, optionally wherein the pressure treatment comprises homogenizing the propagated cultured of the yeast to provide the inactivated yeast composition.

8. The process of any one of claims 5 to 7, wherein:
(a) the yeast composition is as defined in any one of claims 1 to 4;
(b) the yeast comprises a *non-Saccharomyces* yeast, optionally wherein the non-*Saccharomyces* yeast is as defined in claim 4.

9. A yeast composition obtainable or obtained by the process of any one of claims 5 to 8.

10. A method for preventing or limiting the oxidation of an edible or a drinkable product, the method comprising contacting a yeast composition as defined in any one of claims 1 to 4 and 9 with a component of the edible or the drinkable product so as to prevent or limit the oxidation of the component.

11. The method of claim 10, wherein:
(a) the method further comprises fermenting the component, and optionally further comprises contacting the yeast composition with the component before, during and/or after fermentation;
(b) the drinkable product is wine, optionally wherein the component is a wine must and/or the method is for protecting wine or must from oxidative damage.

12. A method of increasing the robustness of a fermentative organism during a fermentation, the method comprises, prior to the fermentation, contacting a yeast composition as defined in any one of claims 1 to 4 and 9 with the fermentative organism to increase the intracellular content of at least one vitamin and/or at least one sterol in the fermentative organism.

13. The method of claim 12, wherein:
(a) the method further comprises fermenting a biomass with the fermentative organism;
(b) the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Yarrowia* sp., *Brettanomyces* sp. or *Candida* sp;
(c) the fermentative organism, prior to the fermentation, is provided in a dried form and is contacted with the yeast composition during rehydration.

14. A method of providing a source of nutrition to a fermentative organism during a fermentation, the method comprises, prior to and/or during the fermentation, contacting a yeast composition as defined in any one of claims 1 to 4 and 9 with the fermentative organism.

15. The method of claim 14, wherein:
(a) the fermentative organism comprises at least one of *Saccharomyces* sp., *Torulaspora* sp., *Metschnikowia* sp., *Pichia* sp., *Lachancea* sp., *Starmerella* sp., *Kluyveromyces* sp., *Yarrowia* sp., *Brettanomyces* sp. or *Candida* sp; and/or
(b) the method comprises adding the yeast composition to a fermentation medium.
